(19)

**Europäisches Patentamt**
**European Patent Office**
**Office européen des brevets**

(11) **EP 4 418 147 A1**

(12) **EUROPEAN PATENT APPLICATION**

(43) Date of publication:
**21.08.2024 Bulletin 2024/34**

(21) Application number: **23156528.4**

(22) Date of filing: **14.02.2023**

(51) International Patent Classification (IPC):
**G06F 16/906** (2019.01)   **G16H 50/20** (2018.01)
**G06N 3/044** (2023.01)   **G06N 3/084** (2023.01)
**G06N 3/0895** (2023.01)   **G06N 3/09** (2023.01)

(52) Cooperative Patent Classification (CPC):
**G06N 3/084; G06F 16/906; G06N 3/044;**
**G06N 3/0455; G06N 3/0895; G06N 3/09;**
**G16H 40/63; G16H 50/20;** G06N 3/0464;
G16H 30/40

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Nokia Technologies Oy**
**02610 Espoo (FI)**

(72) Inventors:
• **DELDARI, Shohreh**
  **Cambridge (GB)**
• **SPATHIS, Dimitrios**
  **Cambridge (GB)**
• **MATHUR, Akhil**
  **London (GB)**
• **MALEKZADEH, Mohammad**
  **Cambridge (GB)**

(74) Representative: **Nokia EPO representatives**
**Nokia Technologies Oy**
**Karakaari 7**
**02610 Espoo (FI)**

(54) **APPARATUS & METHOD FOR GENERATING FEATURE EMBEDDINGS**

(57)     Apparatus comprising means for: obtaining a first data sample and a second data sample; transforming the first data sample into a first feature embedding using a first machine learning model; transforming the second data sample into a second feature embedding using a second machine learning model; and generating a first global representation by masking at least one of: the first feature embedding or the second feature embedding. The apparatus further comprising means for: transforming the first global representation into a third feature embedding using a third machine learning model; and training at least the third machine learning model based on the third feature embedding.

FIG. 13A

**Description**

Field

Various example embodiments relate to an apparatus & a method suitable for generating feature embeddings.

Background

Machine learning models have been used for performing various tasks. One use of machine learning models is to generate inferences/predictions for a specific task based on sensor data. For example, determining a condition of a user based on sensor data that observes/measures the state of the user.

It has been found that using multimodal data (i.e. data that contains different types and contexts) can increase prediction accuracy. An example of multimodal data is a data set that contains image and audio data. Multimodal data is sometimes acquired using different sensors (e.g. a first sensor for obtaining image data and a second sensor for obtaining audio data). It is possible that, in use, data from one of the sensors becomes temporarily unavailable. For example, due to interference in the communication channel with the sensor. In this case, machine learning models that require multimodal data to generate predictions/inferences can suffer a drop in performance.

Summary

According to a first aspect there is provided an apparatus comprising means for: obtaining a first data sample and a second data sample; transforming the first data sample into a first feature embedding using a first machine learning model; transforming the second data sample into a second feature embedding using a second machine learning model; generating a first global representation by masking at least one of: the first feature embedding or the second feature embedding; transforming the first global representation into a third feature embedding using a third machine learning model; and training at least the third machine learning model based on the third feature embedding.

In an example the apparatus is suitable for learning a data compression transform. In an example the third feature embedding is a compressed representation of the first data sample and the second data sample.

In an example the machine learning models are configured to transform an input value to an output value based on a plurality of trainable weights.

In an example a feature embedding is a vector of values that represents information provided at an input using less values. Optionally, the feature embedding is a lower-dimensional representation of the input information. Optionally, the feature embedding is a compressed version of the input data.

In an example the first global representation is a vector of values.

In an example generating a first global representation by masking at least one of: the first feature embedding or the second feature embedding comprises masking at least one of, but not all of: the first feature embedding or the second feature embedding.

In an example masking at least one of: the first feature embedding or the second feature embedding comprises not including the at least one of the first feature embedding or the second feature embedding in the first global representation.

In an example, the first global representation comprises a first position associated with the first feature embedding and a second position associated with the second feature embedding, and wherein masking at least one of the first feature embedding or the second feature embedding comprising setting a corresponding value associated with the first position or the second position equal to a null value (e.g. zero).

In an example the first global representation comprises at least one of: the first feature embedding or the second feature embedding.

In an example the means are further configured for: providing parameters of the third machine learning model to a process after training the third machine learning model. In an example the parameters comprises weights used by the third machine learning model.

In an example the means are further configured for: transmitting parameters of the third machine learning model to a second apparatus after training the third machine learning model.

In an example the third machine learning model is associated with a plurality of weights and wherein training the third machine learning model comprises adjusting the plurality of weights in order to change the value of a metric (e.g. an objective function).

In an example, training at least the third machine learning model based on the third feature embedding comprises: training the first machine learning model, the second machine learning model, and the third machine learning model based on the third feature embedding.

In an example the means are further configured for: transmitting information identifying weights of the first machine learning model, the second machine learning model and the third machine learning model to a second apparatus after training the first machine learning model, the second machine learning model and the third machine learning model.

In an example the first data sample is associated with a first sensor and the second data sample is associated with a second sensor.

In an example the first sensor and the second sensor monitor an industrial process.

In an example the first sensor and the second sensor monitor data associated with a human user. In an example, the first sensor and the second sensor monitor activity of a human user.

In an example the first data sample is associated with a first data mode and the second data sample is associated with a second data mode.

In an example the first data sample comprises a first plurality of data samples, the second data sample comprises a second plurality of data samples, the first feature embedding comprises a first plurality of feature embeddings, the second feature embedding comprises a second plurality of feature embeddings; and wherein: generating the first global representation by masking at least one of: the first feature embedding or the second feature embedding comprises: masking at least one feature embedding in the combination of the first plurality of feature embeddings and the second plurality of feature embeddings.

In an example the first global representation comprises at least one feature embedding from the first plurality of feature embeddings and at least one feature embedding from the second plurality of feature embeddings.

In an example the first global representation does not contain all of the embeddings in the first plurality of feature embeddings and the second plurality of feature embeddings. In an example, the first global representation comprises at least one feature embedding from the first plurality of feature embeddings or the second plurality of feature embeddings.

In an example generating the first global representation by masking at least one feature embedding in the combination of the first plurality of feature embeddings and the second plurality of feature embeddings, comprises: obtaining a threshold value; generating a random number; determining if the random number is greater than the threshold value; and masking a first embedding in the first plurality of feature embeddings in response to determining that the random number is less than the threshold value.

In an example generating the first global representation by masking at least one feature embedding in the combination of the first plurality of feature embeddings and the second plurality of feature embeddings, comprises: adding the first embedding in the first plurality of feature embeddings to the global representation in response to determining that the random number is greater than the threshold value. In an example the threshold value is a masking rate.

In an example generating a random number comprises sampling from a uniform distribution.

In an example the threshold value and the random number have the same range of values.

In an example generating the first global representation by masking at least one feature embedding in the combination of the first plurality of feature embeddings and the second plurality of feature embeddings, comprises: determining a pivot location; determining a position value by sampling from a probability distribution, wherein the mean of the probability distribution is the pivot location; and adding a first embedding from the first plurality of feature embeddings to the first global representation based on the position value.

In an example the position value is associated with an embedding in the first plurality of feature embeddings and wherein adding the first embedding from the first plurality of embeddings comprises identifying the embedding associated with the position value and adding the embedding to the first global representation.

In an example determining a pivot location comprises selecting a value from a range of values.

In an example a first value in the range of values corresponds to a first embedding in the first plurality of feature embeddings and a second value in the range of values corresponds to a second embedding in the first plurality of feature embeddings. In an example the range of values used for the pivot location spans a range equal to a number of feature embeddings in the first plurality of feature embeddings.

In an example a first value in the range of values corresponds to the first plurality of feature embeddings and a second value in the range of values corresponds to the second plurality of feature embeddings. In an example the range of values used for the pivot location spans a range equal to a number of input data sources or input data modes.

In an example the probability distribution is a normal distribution.

In an example the means are further configured for: generating a second global representation by masking at least one of: the first feature embedding or the second feature embedding. transforming the second global representation into a fourth feature embedding using the third machine learning model; and wherein: training at least the third machine learning model based on the third feature embedding comprises: training at least the third machine learning model based on the third feature embedding and the fourth feature embedding.

In an example the first global representation is different to the second global representation.

In an example generating the second global representation by masking at least one of: the first feature embedding or the second feature embedding comprises: obtaining the pivot location; determining a second position value by sampling from the probability distribution; and adding a second embedding from the first plurality of feature embeddings to the second global representation based on the second position value.

In an example training at least the third machine learning model based on the third feature embedding and the fourth feature embedding comprises: determining a value of a first objective function, wherein the first objective function indicates a similarity between the third feature embedding and the fourth feature embedding; and training at least the third machine learning model based on the value of the first objective function.

In an example the third machine learning model is associated with a set of trainable weights and wherein training at least the third machine learning model based on the value of the objective function comprises: modifying the set of trainable weights in order to change the value of the objective function.

In an example training at least the third machine learning model based on the third feature embedding comprises: generating a first prediction using a fourth machine learning model and the first global representation; obtaining a second value associated with the first data sample and the second data sample; determining a value of a second objective function based on the first prediction and the second value; and training at least the third machine learning model based on the value of the second objective function.

In an example the fourth machine learning model is a classifier and the second value is a class label associated with the first data sample and the second data sample.

In an example training at least the third machine learning model based on the value of the second objective function comprises: training the first machine learning model, the second machine learning model, the third machine learning model and the fourth machine learning model based on the value of the second objective function.

In an example the means are further configured for: obtaining a third data sample and a fourth data sample; transforming the third data sample into a fifth feature embedding using the first machine learning model; transforming the fourth data sample into a sixth feature embedding using the second machine learning model; generating a third global representation by combining the fifth feature embedding and the sixth feature embedding; and transforming the third global representation into a seventh feature embedding using the third machine learning model.

In an example transforming the third data sample and transforming the fourth data sample is performed after training at least the third machine learning model.

In an example the means are further configured for: transmitting the third global representation.

In an example the seventh feature embedding is a compressed representation of the third data sample and the fourth data sample.

In an example combining includes concatenating.

In an example the means are further configured for: generating a second prediction using the fourth machine learning model and the third global representation.

In an example the means are further configured for: displaying the second prediction.

In an example the means are further configured for: using the second prediction for controlling an industrial process.

In an example the means are further configured for transmitting the second prediction.

In an example obtaining the first data sample comprises: receiving the first data sample and modifying a value of the first data sample.

In an example modifying the value of the first data sample comprises augmenting the first data value. In an example modifying the value of the first data samples includes adding random noise.

In an example the means comprises: at least one processor; and at least one memory storing instructions that, when executed by the at least one processor, cause the apparatus at least to perform the functionality of any preceding claim.

According to a second aspect there is provided an apparatus comprising: at least one processor; and at least one memory storing instructions that, when executed by the at least one processor, cause the apparatus at least to: obtain a first data sample and a second data sample; transform the first data sample into a first feature embedding using a first machine learning model; transform the second data sample into a second feature embedding using a second machine learning model; generate a first global representation by masking at least one of: the first feature embedding or the second feature embedding; transform the first global representation into a third feature embedding using a third machine learning model; and train at least the third machine learning model based on the third feature embedding.

According to a third aspect there is provided a method comprising: obtaining a first data sample and a second data sample; transforming the first data sample into a first feature embedding using a first machine learning model; transforming the second data sample into a second feature embedding using a second machine learning model; generating a first global representation by masking at least one of: the first feature embedding or the second feature embedding; transforming the first global representation into a third feature embedding using a third machine learning model; and training at least the third machine learning model based on the third feature embedding.

In an example the method is suitable for compressing the first data sample and the second data sample.

In an example the method is a computer implemented method.

In an example training at least the third machine learning model based on the third feature embedding comprises: training the first machine learning model, the second machine learning model, and the third machine learning model based on the third feature embedding.

In an example the first data sample is associated with a first sensor and the second data sample is associated with a second sensor.

In an example the first data sample comprises a first plurality of data samples, the second data sample comprises a second plurality of data samples, the first feature embedding comprises a first plurality of feature embeddings, the second feature embedding comprises a second plurality of feature embeddings; and wherein: generating the first global representation by masking at least one of: the first feature embedding or the second feature embedding comprises: masking at least one feature embedding in the combination of the first plurality of feature embeddings and the second plurality of feature embeddings.

In an example generating the first global representation by masking at least one feature embedding in the combination of the first plurality of feature embeddings and the second plurality of feature embeddings, comprises: obtaining a threshold value; generating a random number; determining if the random number is greater than the threshold value; and masking a first embedding in the first plurality of feature embeddings in response to determining that the random number is less than the threshold value.

In an example generating the first global representation by masking at least one feature embedding in the combination of the first plurality of feature embeddings and the second plurality of feature embeddings, comprises: determining a pivot location; determining a position value by sampling from a probability distribution, wherein the mean of the probability distribution is the pivot location; and adding a first embedding from the first plurality of feature embeddings to the first global representation based on the position value.

In an example the method further comprises: generating a second global representation by masking at least one of: the first feature embedding or the second feature embedding; transforming the second global representation into a fourth feature embedding using the third machine learning model; and wherein: training at least the third machine learning model based on the third feature embedding comprises: training at least the third machine learning model based on the third feature embedding and the fourth feature embedding.

In an example generating the second global representation by masking at least one of: the first feature embedding or the second feature embedding comprises: obtaining the pivot location; determining a second position value by sampling from the probability distribution; and adding a second embedding from the first plurality of feature embeddings to the second global representation based on the second position value.

In an example training at least the third machine learning model based on the third feature embedding and the fourth feature embedding comprises: determining a value of a first objective function, wherein the first objective function indicates a similarity between the third feature embedding and the fourth feature embedding; and training at least the third machine learning model based on the value of the first objective function.

In an example training at least the third machine learning model based on the third feature embedding comprises: generating a first prediction using a fourth machine learning model and the first global representation; obtaining a second value associated with the first data sample and the second data sample; determining a value of a second objective function based on the first prediction and the second value; and training at least the third machine learning model based on the value of the second objective function.

In an example training at least the third machine learning model based on the value of the second objective function comprises: training the first machine learning model, the second machine learning model, the third machine learning model and the fourth machine learning model based on the value of the second objective function.

In an example, the method further comprises: obtaining a third data sample and a fourth data sample; transforming the third data sample into a fifth feature embedding using the first machine learning model; transforming the fourth data sample into a sixth feature embedding using the second machine learning model; generating a third global representation by combining the fifth feature embedding and the sixth feature embedding; and transforming the third global representation into a seventh feature embedding using the third machine learning model.

In an example, the method further comprises: generating a second prediction using the fourth machine learning model and the third global representation.

According to a fourth aspect there is provided a computer program comprising instructions which, when executed by an apparatus, cause the apparatus to perform at least the following: obtaining a first data sample and a second data sample; transforming the first data sample into a first feature embedding using a first machine learning model; transforming the second data sample into a second feature embedding using a second machine learning model; generating a first global representation by masking at least one of: the first feature embedding or the second feature embedding; transforming the first global representation into a third feature embedding using a third machine learning model; and training at least the third machine learning model based on the third feature embedding.

In an example the computer program described above, further comprises instructions, which, when executed by the apparatus, cause the apparatus to perform the method of any preceding method.

According to a fifth aspect there is provided an apparatus comprising means for: obtaining a first data sample and a second data sample; transforming the first data sample into a first feature embedding using a first machine learning model; transforming the second data sample into a second feature embedding using a second machine learning model; generating a first global representation by masking at least one of: the first feature embedding or the second feature embedding; and transforming the first global representation into a third feature embedding using a third machine learning model; wherein: the first machine learning model, the second machine learning model, and the third machine learning model are obtained using the method described above.

According to a sixth aspect there is provided a non-transitory computer readable medium comprising program instructions that, when executed by an apparatus, cause the apparatus to perform at least the following: obtaining a first data sample and a second data sample; transforming the first data sample into a first feature embedding using a first machine learning model; transforming the second data sample into a second feature embedding using a second machine learning model; generating a first global representation by masking at least one of: the first feature embedding or the second feature embedding; transforming the first global representation into a third feature embedding using a third machine learning model; and training at least the third machine learning model based on the third feature embedding.

According to a seventh aspect there is provided an apparatus comprising means for: obtaining information identifying: a first machine learning model; a second machine learning model; and a third machine learning model. The apparatus further comprising means for: obtaining a first data sample and a second data sample; transforming the first data sample into a first feature embedding using the first machine learning model; transforming the second data sample into a second feature embedding using the second machine learning model; generating a first global representation by combining the first feature embedding and the second feature embedding; and transforming the first global representation into a third feature embedding using the third machine learning model.

In an example the apparatus further comprises means for: obtaining a fourth machine learning model and generating a first prediction using the fourth machine learning model and the first global representation.


Brief Description of the Drawings


Some examples will now be described with reference to the accompanying drawings in which:

Fig. 1 shows a multi-modal machine learning system according to an example;
Fig. 2 shows a first machine learning architecture 200 used during inference according to an example;
Fig. 3 shows a method of inference accordance to an example;
Fig. 4 shows a second machine learning architecture 400 used during self-supervised training according to an example;
Fig. 5 shows a method of training a first part of the first machine learning architecture 200 according to an example;
Fig. 6 shows random patch selection according to an example;
Fig. 7A shows locality-aware patch selection according to an example;
Fig. 7B shows an example of spatial locality-aware masking according to an example;
Fig. 8 shows an illustration of the terms used in an objective function according to an example;
Fig. 9 shows a third machine learning architecture 900 used during supervised training according to an example;
Fig. 10 shows a method of training a second part of the first machine learning architecture 200 according to an example;
Fig. 11 shows a first method of deploying the first machine learning architecture 200 in the multi-modal machine learning system 100 according to an example;
Fig. 12 shows a second method of deploying the first machine learning architecture 200 in the multi-modal machine learning system 100 according to an example;
Fig. 13A shows a method of training at least the third machine learning model according to an example;
Fig. 13B shows a performance comparison according to an example;
Fig. 14 shows an illustration of a fully connected (artificial) Neural network according to an example;
Fig. 15 shows an implementation of the first apparatus according to an example.


In the figures same reference numerals denote same functionality/components.


Detailed Description


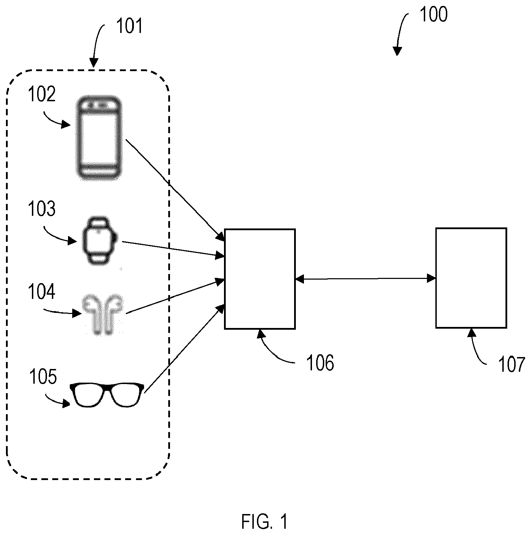

Fig. 1 shows a multi-modal machine learning system according to an example. More specifically, Fig. 1 shows a multi-modal machine learning system 100 comprising a first set of sensors 101. The first set of sensors 101 shown in Fig. 1 comprises a first sensor 102, a second sensor 103, a third sensor 104 and a fourth sensor 105.

The methods described herein will be discussed in relation to an example where the number of sensors, $M$, in the first set of sensors 101 equals 4 (i.e. $M = 4$). However, for the avoidance of any doubt, it is emphasized that in other examples the number of sensors, $M$, takes any value greater than or equal to two.

Each sensor in the first set of sensors 101 is configured to observe/measure a property of an environment. At least two sensors in the first set of sensors 101 are configured to observe different properties of the environment. Or put in other words, at least two sensors in the first set of sensors 101 are configured to observe/measure different data modes. Consequently, the data from the first set of sensors 101 is multimodal data because it comprises data that spans different types and contents.

In the example of Fig. 1 the first sensor 102 is implemented in a smartphone and measures motion data, the second sensor 102 is implemented in a smart watch and captures medical data (e.g. heart rate etc.), the third sensor 103 is implemented in a set of earphones and measures audio data, and the fourth sensor 104 is implemented in a pair of smart glasses and captures image data. Consequently, the data from the first set of sensors 101 is multimodal data because it comprises different types of data (e.g. motion data, medical data, audio data, and image data).

Each sensor in the first set of sensors 101 is communicatively coupled (either directly or indirectly) to a first apparatus 106. The first apparatus 106 is also referred to as "the host device". Optionally, the multi-modal machine learning system 100 also comprises a second apparatus 107. The second apparatus 107 is also referred to as "the server". In this example, the first apparatus 106 is communicatively coupled to the second apparatus 107.

In an example the first apparatus 106 comprises a sensor in the first set of sensors 101. In one example the first apparatus 106 is a User Equipment (UE) device (e.g. a smart phone) that also implements the first sensor 102.

The functionality of the first apparatus 106 will be discussed in more detail below. However, in brief, the first apparatus 106 is configured to: 1) train at least part of a machine learning architecture for the purpose of performing a specific task based on data from the first set of sensors 101; and/or 2) generate predictions/inferences based on the trained machine learning architecture and the data generated by the first set of sensors 101.

The machine learning architecture that the first apparatus 106 uses to generate predictions/inferences will now be discussed in detail.

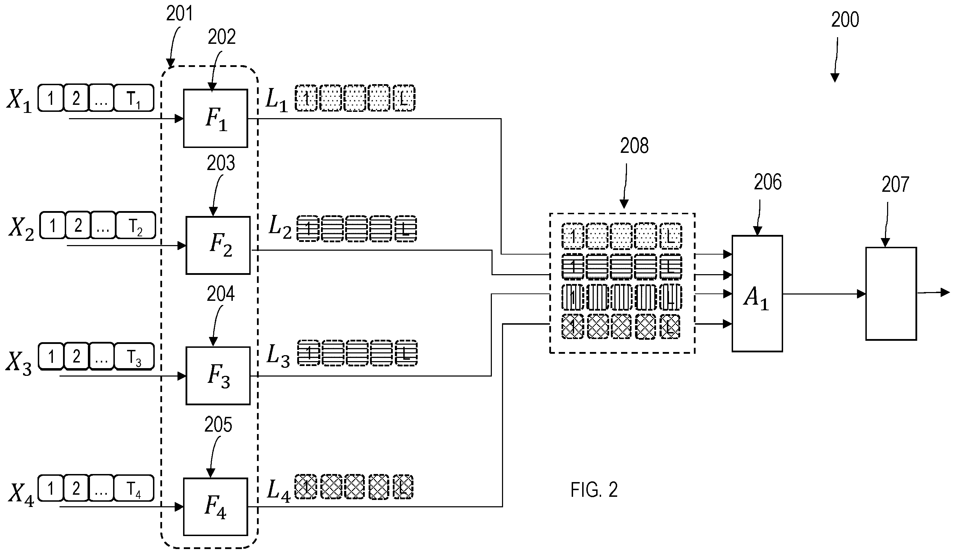

Fig. 2 shows a first machine learning architecture 200 used during inference according to an example. In the present application, the term machine learning architecture is used to describe a collection of one or more processes that implement/use machine learning to perform a particular task. In an example the first machine learning architecture 200 is implemented as a series of instructions in computer program code. The components of the first machine learning architecture 200 will be discussed first before discussing how these components are used for inference.

The first machine learning architecture 200 comprises a set of feature extractors 201, a first aggregator 206, and a classifier (or regressor). The classifier is implemented by a fourth machine learning model 207.

The set of feature extractors 201 comprises a feature extractor for each sensor in the first set of sensors 101. Consequently, each feature extractor in the set of feature extractors 201 can also be referred to as a "modality-specific" feature extractor, since each sensor in the first set of sensors 101 generates a different data mode. A feature extractor may also be referred to as a feature encoder, and the set of feature extractors may be referred to as the set of feature encoders. In the example shown in Fig. 2, the set of feature extractors 201 comprises a first feature extractor 202, $F_1$, a second feature extractor 203, $F_2$, a third feature extractor 204, $F_3$, and a fourth feature extractor 205, $F_4$.

Each feature extractor in the set of feature extractors 201 is configured to generate a representation of the input data that conveys the information contained within the input data while reducing the number of resources required to convey this information. Or put in other words, each feature extractor is configured to reduce the amount of redundant data in the input data.

In an example, each feature extractor in the set of feature extractors 201 comprises a machine learning model that is configured to convert input data into a local embedding (i.e. an output representation) based on one or more trainable weights. In particular, each feature extractor in the set of feature extractors is configured to convert the input data into a local embedding based on a mathematical function, where the properties of the mathematical function are learnt. In an example the machine learning model comprises an (artificial) neural network. Specific details of the machine learning models used by each feature extractor in the set of feature extractors 201 will be discussed in more detail below. In an example, different feature extractors in the set of feature extractors 201 use structurally different machine learning models.

In the example of Fig. 2 the first feature extractor 202, $F_1$, is configured to transform a first set of input data samples associated with the first sensor 102, $X_1[1,2, ..., T_1]$, into a first set of local embeddings, $L_1[1,2, ... , L]$, based on a first set of trainable weights, $W_1$. In an example, the first feature extractor 202, $F_1$, comprises a first machine learning model.

The second feature extractor 203, $F_2$, is configured to transform a second set of input data samples associated with the second sensor 103, $X_2[1,2, ... , T_2]$, into a second set of local embeddings, $L_2[1,2, ... , L]$, based on a second set of trainable weights, $W_2$. In an example, the second feature extractor 203, $F_2$, comprises a second machine learning model.

The third feature extractor 204, $F_3$, is configured to transform a third set of input data samples associated with the third sensor 104, $X_3[1,2, ... , T_3]$, into a third set of local embeddings, $L_3[1,2, ...,L]$, based on a third set of trainable weights, $W_3$. In an example, the third feature extractor 204, $F_3$, comprises a fifth machine learning model.

The fourth feature extractor 205, $F_4$, is configured to transform a fourth set of input data samples associated with the fourth sensor 104, $X_4[1,2, ...,T4]$, into a fourth local embedding, $L_4[1,2, ..., L]$, based on a fourth set of trainable weights, $W_4$. In an example, the fourth feature extractor 205, $F_4$, comprises a sixth machine learning model.

In an example, the number of time samples in the sets of input data samples are equal (i.e. $T_1 = T_2 = T_3 = T_4$). In another example, the number of samples in the at least two sets of input data samples are different (e.g. $T_1 \neq T_2$). In an example, the number of samples in the set of input data samples (e.g. $T_1$) is selected based on the type of sensor and the task that the input data is being used for. In an example, the number of samples is selected such that there is enough data to learn good discrimination patterns for all classes.

In an example, each of the feature extractors in the set of feature extractors 201 are configured to output feature embeddings of the same length (e.g. L). As will be discussed in more detail below, in one example the feature extractors use a sequence model (e.g. a Recurrent Neural Network) that takes an input of user-specific length (e.g. $T_1$, $T_2$, $T_3$, $T_4$) and generates an output of fixed length (e.g. L).

The outputs of each feature extractor in the set of feature extractors 201 (i.e. the sets of local embeddings) are provided to the first aggregator 206, $A_1$. In an example the sets of local embeddings are combined (e.g. by concatenation) into a global representation 208 that is subsequently provided as an input to the first aggregator 206, $A_1$.

The first aggregator 206, $A_1$, is also a feature extractor in the sense it is configured to transform the information contained in the input (i.e. the sets of local embeddings) into a lower dimensional representation that preserves the information contained in the input. However, unlike the feature extractors in the set of feature extractors 201 that generate modality-specific local embeddings, the aggregator 206, $A_1$, generates a global embedding that considers the dependencies between the dimensions of the sets of local embeddings. For example, the aggregator 206, $A_1$, generates a global embedding that takes account of the temporal (i.e. across time) and the spatial (i.e. across sensors) dependencies in the input.

The first aggregator 206, $A_1$, is configured to generate a first global embedding, $e_i^1$, based on the global representation 208 and a fifth set of trainable weights, $W_5$. In an example the first aggregator 206 comprises a third machine learning model that is configured to convert input data (i.e. the global representation 208 comprising the sets of local embeddings) into a global embedding (i.e. an output representation) based on one or more trainable weights. In particular, the first aggregator 206 is configured to convert the input data into a global embedding based on a mathematical function, where the properties of the mathematical function are learnt. In an example the third machine learning model used by the first aggregator 206 comprises an (artificial) neural network.

The output of the first aggregator 206, $A_1$, is a first global embedding, $e_i^1$. The first global embedding, $e_i^1$, is also referred to as a first latent representation. In the first machine learning architecture 200 of Fig. 2, the first global embedding, $e_i^1$, is provided as an input to the fourth machine learning model 207.

The fourth machine learning model 207 is configured to generate a prediction/inference based on the first global embedding and a sixth set of trainable weights, $W_6$. In particular, the fourth machine learning model 207 is configured to generate a prediction/inference based on a mathematical function, where the properties of the mathematical function are learnt. In an example the fourth machine learning model 207 comprises an (artificial) neural network.

The properties (e.g. the structure and the output) of the fourth machine learning model 207 depends on the task being performed by the first machine learning architecture 200. In an example where the first machine learning architecture 200 is used for a classification task (i.e. predicting a class label that represents the input data), the output of the fourth machine learning model 207 comprises a prediction of the class label associated with the input data. In another example where the first machine learning architecture 200 is used for a regression task (i.e. predicting a value of a variable associated with the input data) the output comprises a prediction of the variable value.

The methods described herein will be introduced with reference to an example scenario where the first machine learning architecture 200 is used to predict whether a user (e.g. that is wearing the sensors in the first set of sensors 101) has fallen over. This information is of particular value for managing elderly and frail patients. As a result, the fourth machine learning model 207 is configured for classification and the output of the fourth machine learning model 207 comprises an indication of whether or not the user has fallen over.

A method of inference performed by the first apparatus 106 using the first machine learning architecture 200 will now be discussed in detail.

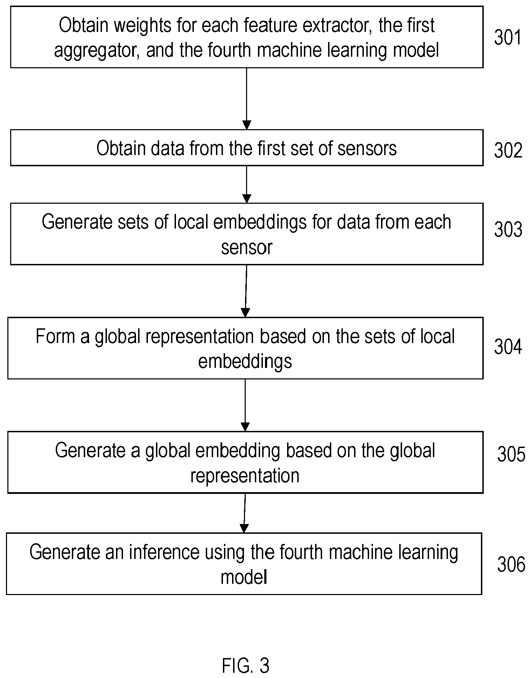

Fig. 3 shows a method of inference accordance to an example. The method begins in step 301.

In step 301 weights for: 1) each of the feature extractors in the set of feature extractors 201; 2) the first aggregator 206; and 3) the fourth machine learning model 207 are obtained. More specifically, when the method of Fig. 3 is used with the first machine learning architecture 200, step 301 comprises obtaining: the first set of trainable weights, $W_1$, the second set of trainable weights, $W_2$, the third set of trainable weights, $W_3$, the fourth set of trainable weights, $W_4$, the

fifth set of trainable weights, $W_5$, and the sixth set of trainable weights, $W_6$.

In an example, at least some of the trainable weights are obtained by retrieving the weights from a memory (e.g. a volatile or non-volatile memory of the first apparatus 106). In another example at least some of the trainable weights are obtained by receiving the weights from an external apparatus (e.g. a server). In an example the weights obtained in step 301 are generated by using the methods of training the first machine learning architecture 200 discussed further below. After obtaining the trainable weights in step 301, the method proceeds to step 302.

In step 302 data is obtained from the set of sensors 101. The data obtained in step 302 is unlabelled. Or put in other words, the data 302 does not contain an indication of the class label. In an example the data comprises: the first set of input data samples $X_1[1,2, ... , T_1]$ associated with the first sensor 102, the second set of input data samples $X_2[1,2, ...,T_2]$ associated with the second sensor 103, the third set of input data samples $X_3[1,2, ...,T_3]$ associated with the third sensor 104, and the fourth set of input data samples $X_4[1,2, ...,T4]$ associated with the fourth sensor 105.In an example, the data is obtained in step 302 by transmitting a request for data to each sensor in the set of sensors 101.

In an example obtaining the first set of input samples comprises receiving data from the first sensor and applying a sliding window to the received samples to obtain the first set of input samples. In an example the sliding windows for a given sensor have an overlap. After obtaining the data in step 302 the method proceeds to step 303.

In step 303 sets of local embeddings are generated for data from each of the sensors in the set of sensors 101. More specifically, in step 303: 1) a first set of local embeddings, $L_1[1,2, ...,L]$, is generated based on the first set of input data samples $X_1[1,2, ... , T_1]$ and the first set of weights. $W_1$; 2) a second set of local embeddings, $L_2[1,2, ..., L]$ is generated based on the second set of input data samples $X_2[1,2, ..., T_2]$ and the second set of weights, $W_2$; 3) a third set of local embeddings, $L_3[1,2, ..., L]$ is generated based on the third set of input data samples $X_3[1,2, ... , T_3]$ and the third set of weights, $W_3$; and 4) a fourth set of local embeddings, $L_4[1,2, ..., L]$ is generated based on a fourth set of input data samples $X_4[1,2, ..., T_4]$ and the fourth set of weights, $W_4$. The method proceeds to step 304.

In step 304 a global representation of the sets of local embeddings is formed. In an example forming the global representation comprises concatenating the sets of local embeddings into a single data structure (e.g. a single vector). The global representation has size $M \times L$, where $M$ is the number of sensors in the set of sensors 101 and $L$ is the number of local embeddings in the set of local embeddings. The method proceeds to step 305.

In step 305 a global embedding is generated based on the global representation. In particular, in step 305 a global embedding, is generated by inputting the first global representation into the first aggregator 206 that is configured according to the fifth set of trainable weights, $W_5$. Or put in other words, in step 305 the global representation is transformed, using a mathematical transform, into a global embedding that represents the information contained in the global representation with fewer dimensions, where the properties of the mathematical transform are characterised, at least in part, by the fifth set of trainable weights, $W_5$. After generating the global embedding in step 305, the method proceeds to step 306.

In step 306 an inference/prediction is generated using the fourth machine learning model 207. In particular, in step 306, the fourth machine learning model 207 transforms the input (e.g. the global embedding) into information identifying a class associated with the data provided at the input of the first machine learning architecture. In particular, the fourth machine learning model 207 transforms the input (e.g. the global representation) into the inference/prediction based on the sixth set of weights, $W_6$. After completing step 306 an inference/prediction is obtained.

In the example use case, the output of step 306 comprises an indication of whether or not a user wearing the first of sensors has fallen over. In order to generate this prediction, the first machine learning architecture combines data from multiple different sources/modes (e.g. sound data, image data, accelerometer data).

As discussed above, during inference (specifically in step 304), the data from the multiple different modes is combined (or "fused") in order to generate a global embedding that is subsequently used for the classification task. However, data from one or more sensors in the set of sensors 101 may be temporarily unavailable in use. For example, some of the sensors in the first set of sensors 101 may not be active at the same time because, for example, one of the sensors may have run out of battery or a user may not be wearing the device containing the sensor. The unavailability of input data can have a negative impact on the performance of a machine learning model that uses multimodal data to generate inferences/predictions.

As will be appreciated from the description below, using the methods described herein during training enables the generation of a global embedding that is more robust to missing input data (e.g. by accurately representing the state of the system being observed by the first set of sensors 101 even when some of the input data is missing). This has the effect of enabling higher prediction/inference accuracy because a more accurate representation of the system state at the input will produce a more accurate prediction/inference.

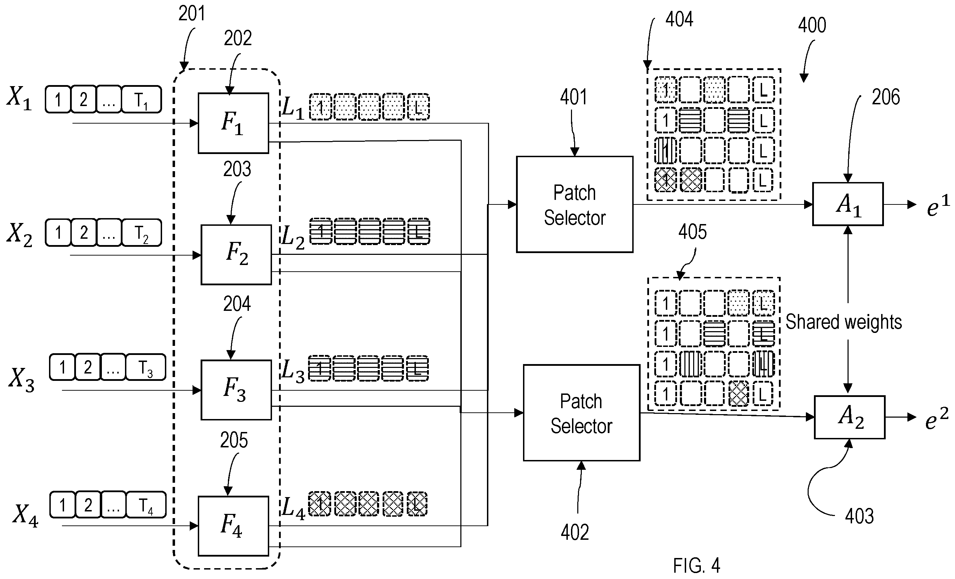

Fig. 4 shows a second machine learning architecture 400 used during self-supervised training according to an example. In particular, Fig. 4 shows a second machine learning architecture 400 that is used to train part of the first machine learning architecture 200. Those parts being the feature extractors in the set of feature extractors 201 and the first aggregator 206. In Fig. 4 same reference numerals as Fig. 2 are used to represent same components. As a result, a detailed discussion of their functionality will be omitted for the sake of brevity.

The second machine learning architecture 400 comprises the set of feature extractors 201, which comprises the first feature extractor 202, $F_1$, the second feature extractor 203, $F_2$, the third feature extractor 204, $F_3$, and the fourth feature extractor 205, $F_4$. The second machine learning architecture 400 further comprises: a first patch selector 401, a second patch selector 402, the first aggregator 206 and a second aggregator 403.

In the second machine learning architecture 400 the outputs of each feature extractor in the set of feature extractors 201 (i.e. the sets of local embeddings) are inputted into first patch selector 401. Similarly, the outputs of each feature extractor in the set of feature extractors 201 (i.e. the sets of local embeddings) are inputted into second patch selector 402.

Each patch selector (i.e. the first patch selector 401 and second patch selector 402) is configured to generate a global representation by combining the sets of local embeddings provided at the input. In an example, each patch selector is configured to generate the global representation by combining the sets of local embeddings from each feature extractor and removing at least one local embedding from the combination. Optionally, the at least one local embedding that is removed is randomly selected.

Consequently, the first patch selector 401 is configured to output a first global representation 404 comprising some, but not all, of the local embeddings in the sets of local embeddings. Similarly, the second patch selector 402 is configured to output a second global representation 405 comprising some, but not all, of the local embeddings in the sets of local embeddings. The second global representation is also referred to as a second latent representation. As will be discussed in more detail below the first global representation 404 and the second global representation 405 are generated from the same input data (i.e. the sets of local embeddings). However, the output representations will be different (with a high likelihood). For example, due to the use of a random variable in the masking process. This has the effect of generating two global representation that represent the same underlying data in different ways. In an example, the dimensions of the first global representation 404 and the second global representation 405 are $M \times L$, where $M$ is the number of input data sources, which equals 4 (i.e. $M = 4$) in the example of Fig. 1, and $L$ is the embedding size of the feature extractors (e.g. the number of local embeddings in a set of local embeddings).

In an example, the embedding size (i.e. the output size) of the first feature extractor 202, $F_1$, is different to the embedding size of the second feature extractor 203, $F_2$. This could occur, for example, when the first feature extractor 202, $F_1$, extracts temporal features (e.g. features which are associated with the temporal behaviour of a sensor) and the second feature extractor 203, $F_2$, extracts features associated with another dimension (e.g. spatial features). In this example, the dimension of the first global representation 404 and the second global representation 405 are $M \times L_{largest}$, where $L_{largest}$ is the largest number of output embeddings in the sets of local embeddings. In an example, where the number of local embeddings associated with a modality is less than $L_{largest}$, the corresponding row of the global representation is made up by padding (e.g. with null values).

The first global representation 404 is provided as an input to the first aggregator 206, $A_1$. Similarly, the second global representation 405 is provided as an input to the second aggregator 403, $A_2$.

As discussed above, the first aggregator 206, $A_1$, is configured to generate a first global embedding, $e^1$, based on the input and a fifth set of trainable weights, $W_5$. In an example the first aggregator 206 comprises a third machine learning model that is configured to convert input data into a global embedding (i.e. an output representation) based on one or more trainable weights. In particular, the first aggregator 206 is configured to convert the input data into a global embedding based on a mathematical function, where the properties of the mathematical function are learnt. In an example the third machine learning model used by the first aggregator 206 comprises an (artificial) neural network.

The second aggregator 403 is configured to perform the same functionality as the first aggregator 206. However, it will be appreciated that the second aggregator 403 has different input data. More specifically, the second aggregator 403, $A_2$, is configured to generate a second global embedding, $e^2$, based on the input (i.e. the second global representation 405) and the fifth set of trainable weights, $W_5$ (i.e. the second aggregator 403 uses the same weights as the first aggregator 206). In an example the second aggregator 403 comprises a machine learning model that is configured to convert input data into a global embedding (i.e. an output representation) based on one or more trainable weights. In particular, the second aggregator 403 is configured to convert the input data into a global embedding based on a mathematical function, where the properties of the mathematical function are learnt. In an example the machine learning model used by the second aggregator 403 comprises an (artificial) neural network.

The method of training the feature extractors in the first set of feature extractors 201, and the first aggregator 206 will now be discussed in detail.

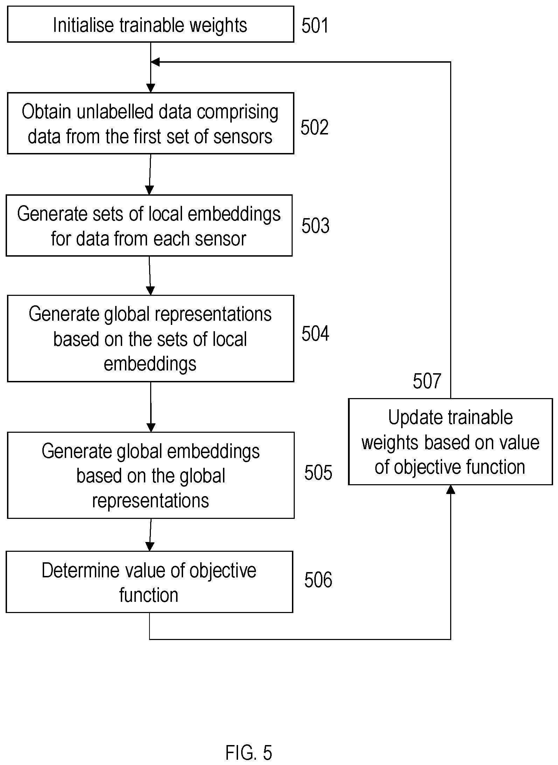

Fig. 5 shows a method of training a first part of the first machine learning architecture 200 according to an example. In particular, the method of Fig. 5 is used for training the feature extractors in the set of feature extractors 201 and the first aggregator 206. In this context, training means learning parameters that could be used by the components during inference. The method begins in step 501.

In step 501 the trainable weights are initialised. In an example the weights of each feature extractor in the set of feature extractors 201 (e.g. $W_1$, $W_2$, $W_3$ and $W_4$) are randomly initialised. Similarly, in step 501 the weights used by the first aggregator 206 (which are also shared with the second aggregator 403), i.e. the fifth set of weights, $W_5$, are

randomly initialised. The method proceeds to step 502.

In step 502 unlabelled data is obtained from each sensor in the first set of sensors 101. In an example the unlabelled data comprises: the first set of input data samples $X_1[1,2, ... , T_1]$ associated with the first sensor 102, the second set of input data samples $X_2[1,2, ... , T_2]$ associated with the second sensor 103, the third set of input data samples $X_3[1,2, ... , T_3]$ associated with the third sensor 104, and the fourth set of input data samples $X_4[1,2, ... , T4]$ associated with the fourth sensor 105. In this context, unlabelled data means data that does not include an associated class label.

Optionally, step 502 comprises applying a sliding window to a plurality of data samples to obtain the sets of input samples.

Optionally, step 502 also comprises a data augmentation step (not shown). In an example, random augmentations are applied to one or more of the sets of input samples, where the augmentation is selected from a set of signal transformations, *T.* Advantageously, applying augmentations to the input data increases diversity in the input space and improves training. In an example, the set of signal transformations, *T* comprises signal transformations that have been found to be effective for the particular application that the first machine learning architecture 200 is being used for.

In an example, the set of signal transformations, *T*, comprises time domain transformations and/or frequency domain transformation. In an example the time domain transformation comprises one or more of:

- "Noise" - Add a randomly generalized noise signal in the time domain.
- "Scale" - Amplify the signal with a randomly generated distortion.
- "Shuffle" - Randomly permute the samples of the signal.
- "Resample" - Resample the signal to a different sampling frequency.
- "Negate" - Multiply the value of the signal by a factor of - 1.

In an example the frequency domain transformations comprise one or more of:

- "hfc" - split the low and high frequency components of the signal and reserve the high frequency components
- "lfc" - split the low and high frequency components of the signal and reserve the low frequency components
- "ap_p" - perturb the amplitude and phase values of a randomly selected segment of the frequency response of the signal.

In an example, the unlabelled data is obtained by transmitting a request for data to each sensor in the first set of sensors 101. In another example the unlabelled data is obtained from a memory containing data recorded by the first set of sensors 101 at a previous time. After obtaining the unlabelled data the method proceeds to step 503.

In step 503 sets of local embeddings are generated for data from each of the sensors in the first set of sensors 101. More specifically, in step 503: 1) a first set of local embeddings, $L_1[1,2, ... , L]$, is generated based on the first set of input data samples $X_1[1,2, ... , T_1]$ and the first set of weights. $W_1$; 2) a second set of local embeddings, $L_2[1,2, ... , L]$ is generated based on the second set of input data samples $X_2[1,2, ... , T_2]$ and the second set of weights, $W_2$; 3) a third set of local embeddings, $L_3[1,2, ... , L]$ is generated based on the third set of input data samples $X_3[1,2, ... , T_3]$ and the third set of weights, $W_3$; and 4) a fourth set of local embeddings, $L_4[1,2, ...,L]$ is generated based on a fourth set of input data samples $X_4[1,2, ..., T_4]$ and the fourth set of weights, $W_4$. The method proceeds to step 504.

In step 504 global representations are generated based on the sets of local embeddings. In particular, in step 504 global representations are generated by masking (i.e. discarding) one or more local embeddings. In an example, a first global representation 404 and a second global representation 405 are obtained by separately masking the sets of local embeddings. In the second machine learning architecture 400, step 504 is performed by the first patch selector 401 (to generate the first global representation 404) and the second patch selector 402 (to generate the second global representation 405).

Two different approaches to patch selection are described herein. These being: 1) random selection; and 2) locality-aware selection. However, it will be appreciated that other approaches to masking one or more local embeddings could also be used in step 504.

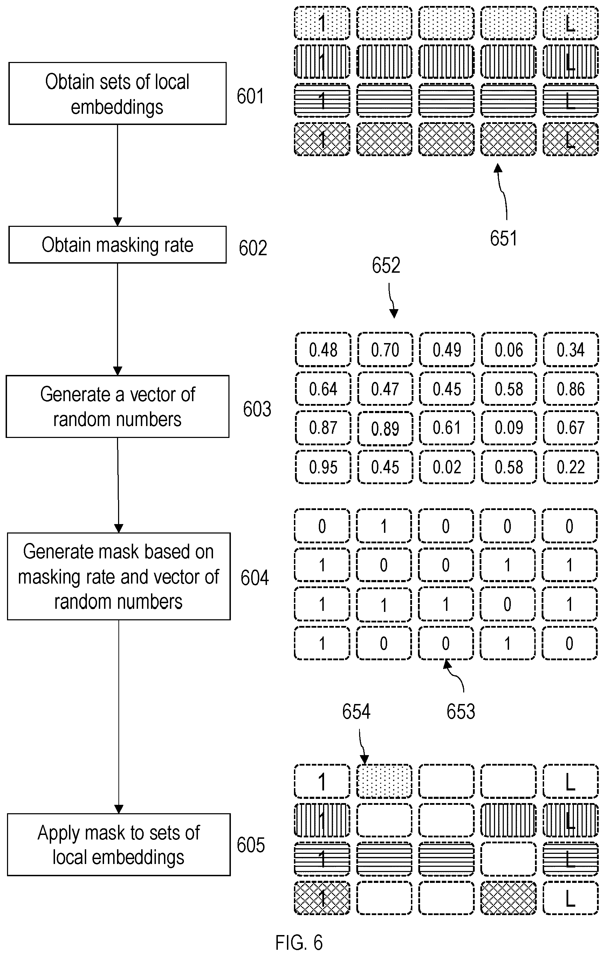

Fig. 6 shows random patch selection according to an example. The method begins in step 601. In step 601 the sets of local embeddings are obtained. A first illustration 651 shows the sets of local embeddings obtained in step 601 in an example. The method proceeds to step 602.

In step 602 the masking rate is obtained. In an example the masking rate is a parameter specified as part of the training methods. In an example the masking rate takes a value between 0 and 1. The masking rate indicates an amount of masking that is to be applied to the sets of local embeddings. In an example the masking rate indicates a fraction or percentage of the sets of local embeddings that are to be masked. After obtaining the masking rate the method proceeds to step 603.

In step 603 a vector of random numbers is generated. In an example, the vector has the same size and dimensions as the sets of local embeddings obtained in step 601. In an example, the random numbers are generated by sampling from a uniform distribution, optionally between 0 and 1. A second illustration 652 shows the vector of random numbers generated in step 603 in an example. The method proceeds to step 604.

In step 604 a mask is generated based on the masking rate and the vector of random numbers. In example, the vector of random numbers is compared to the masking rate (which takes a value between 0 and 1). If the random number in the vector is greater than the masking rate, then a '1' (indicating that the local embedding is to be kept) is added to the corresponding position (i.e. row and column) in the mask vector. If the random number in the vector is less than the masking rate, then a '0' (indicating that the embedding is to be discarded) is added to the corresponding position in the mask vector. It will be appreciated that the mask vector has the same size and dimensions as the vector of random numbers. A third illustration 653 shows the mask according to an example. After obtaining the mask in step 604 the method proceeds to step 605.

In step 605 the mask generated in step 604 is applied to the sets of local embeddings obtained in step 601. In this case, the mask is logically ANDed with the sets of local embeddings. Or put in other words, if the value at a position in the mask vector is '1', then the local embedding at the corresponding position in the input sets of local embeddings is added to the output set of local embeddings in that same position. Alternatively, if the value of the mask vector at a given position (e.g. a row and column value) is '0', then the output set of local embeddings at that position is set to a null value (e.g. zero). A fourth illustration 654 shows the output set of local embeddings after masking has been applied in an example.

The above example describes the applying masking to the local embeddings in parallel. This has the advantage of improved efficiency. However, in other examples, masking is applied serially (e.g. obtain a local embedding associated with a position in the sets of local embeddings, generate a random number from a uniform distribution, determine if random number is greater than masking threshold, if greater add the local embedding to the output set at the position, if not add a null value to the output set at that position, repeat for all positions in the sets of local embeddings).

In an example, the first patch selector 401 and the second patch selector 402 use random masking according to the method of Fig. 6. In this example, both the first patch selector 401 and the second patch selector 402 share the same masking rate.

In this case the first patch selector 401 and the second patch selector 402 implement separate random processes. As a result, it is possible for the first global representation 404 and the second global representation 405 to contain different local embeddings (i.e. there could be no shared local embeddings in the two global representations). It has been found that training is improved when there are some common local embeddings in the global representations.

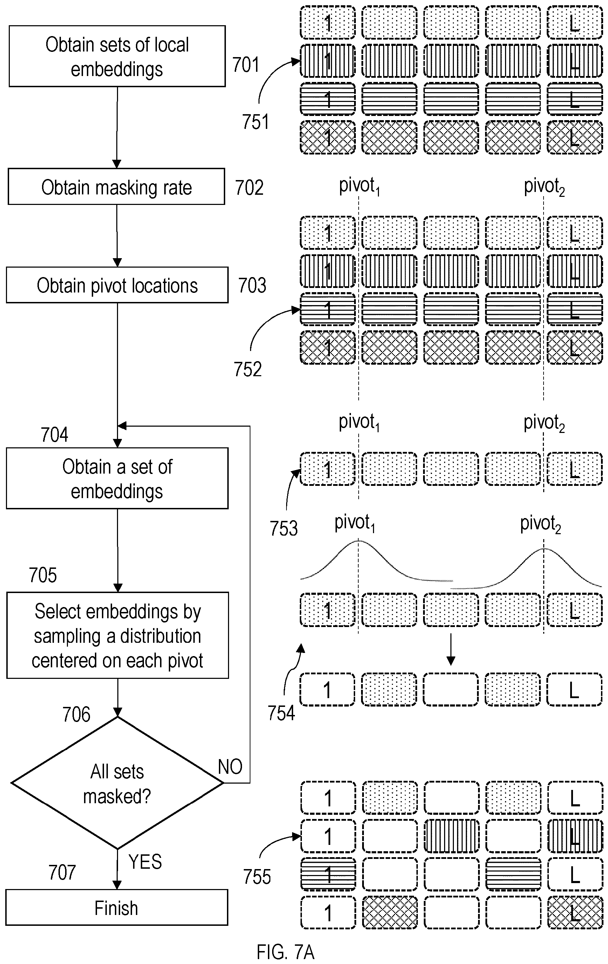

Fig. 7A shows locality-aware patch selection according to an example. The method begins in step 701. In step 701 the sets of local embeddings are obtained. A fifth illustration 751 shows the sets of local embeddings obtained in step 701 in an example. The method proceeds to step 702.

In step 702 a masking rate is obtained. The masking rate indicates an amount of masking that is to be applied to the input sets of local embeddings. The method proceeds to step 703.

In step 703 pivot locations are obtained. In an example, a pivot location is an anchor (i.e. a location/position) in the sets of embeddings for use during subsequent sampling.

In the example shown in Fig. 7A temporal locality-aware masking is applied. In this case, the pivot has a constant position in the temporal direction. For example, Fig. 7A shows a sixth illustration 752 where an example illustration of a first pivot, $pivot_1$, and a second pivot, $pivot_2$, are superimposed over an illustration of the sets of local embeddings. In this example the first pivot, $pivot_1$, is located at $l = 1$, where $l$ takes a value between 0 and $L$. In the example sixth illustration 752 the second pivot, $pivot_2$, is located at $l = 4$.

Since temporal masking is used in this example, the position of the pivots in the spatial dimension (i.e. across sensors) does not change. For example, the first pivot, $pivot_1$, for embeddings associated with the first sensor 102 is located at $l = 1$. Similarly, the first pivot, $pivot_1$, for embeddings associated with the second sensor 103 is located at $l = 1$.

In an example, the pivot locations are obtained in step 703 by sampling $n$ times from a normal distribution between [0, T], where T is the number of local embeddings in the sets of local embeddings and $n$ is the number of pivots. After obtaining the pivot locations the method proceeds to step 704. In another example the pivot locations are predetermined and/or obtained from another process.

In step 704 a set of local embeddings is obtained. A seventh illustration 753 shows a set of local embeddings with the first pivot, $pivot_1$, and the second pivot, $pivot_2$, superimposed thereon. The method proceeds to step 705.

In step 705 embeddings from the set of local embeddings are selected by sampling a probability distribution that is centered on each of the pivots (e.g. has a mean corresponding to the pivot location). For example, the first pivot, $pivot_1$, is associated with a first probability distribution. A value is sampled from the first probability distribution. The sampled value is converted to a local embedding index. The local embedding index indicates local embeddings that are selected. Local embeddings not selected are masked (e.g. set to a null value such as zero). For example, an eighth illustration 754 shows the second and the fourth local embeddings being selected, while the first, third and fifth local embeddings are masked.

In an example the probability distribution is a normal distribution. In another example the probability distribution is a uniform distribution.

The number of samples taken from each probability distribution depends on the masking rate obtained in step 702. For example, in the case that the probability distribution is a normal distribution, the number of samples is determined in a similar way to random sampling (e.g. using the masking rate as a threshold for masking samples).

After selecting embeddings for one set of local embeddings, the method proceeds to step 706.

In step 706, it is determined whether all the sets of local embeddings have been masked (i.e. whether steps 704 and 705 have been completed for each set of local embeddings). If it is determined in step 706 that all of the sets have been masked then the method proceeds to step 707 where the method of locality-aware masking finishes. A nineth illustration 755 shows an example of the sets of local embeddings after masking. If it is determined in step 706 that all of the sets have not been masked, then a new set of embeddings is selected from the sets of local embeddings obtained in step 701 and the method repeats steps 704 and 705 for the new set of embeddings.

In the example of Fig. 7A, temporal locality-aware masking is applied (i.e. the pivot locations vary in the temporal dimension, but not the spatial dimension). In another example spatial locality-aware masking is applied.

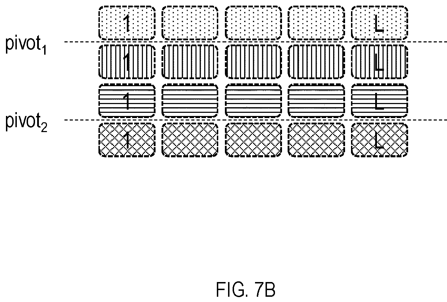

Fig. 7B shows an example of spatial locality-aware masking according to an example. In spatial locality-aware masking the pivots (e.g. the first pivot, $pivot_1$, and the second pivot, $pivot_2$) have positions/values that are fixed in the spatial dimension for each set of embeddings.

For example, In the example of Fig. 7B, the first pivot, $pivot_1$, is located at $m = 1$, and the second pivot is located at $m = 3$, where $m$ takes a value between 0 and $M$ ($M$ being the number of sensors in the set of sensors 101). When spatial locality-aware masking is used, the same method as described in relation to Fig. 7A is used.

In an example, the first patch selector 401 and the second patch selector 402 use locality-aware masking (either temporal or spatial) according to the method of Fig. 7A and 7B. In this example, both the first patch selector 401 and the second patch selector 402 share the same masking rate and also share the same pivot locations. Using the same pivot locations results in two global representations that likely share some (but not all) of the non-masked local embeddings. This has been found to be advantageous for training the set of feature extractors 201 and the aggregator 206.

Returning to Fig. 5. After completing step 504 at least two different global representations (i.e. the first global representation 404 and the second global representation 405) containing different local embeddings are obtained. After completing step 504, the method proceeds to step 505.

In step 505 global embeddings are generated based on the global representations generated in step 504. In step 505 a first global embedding, $e^1$, is generated by inputting the first global representation 404 into the first aggregator 206 that is configured according to the fifth set of trainable weights, $W_5$. Similarly, in step 505 a second global embedding, $e^2$, is generated by inputting the second global representation 405 into the second aggregator 403 that is configured according to the fifth set of trainable weights, $W_5$. After completing step 505 the method proceeds to step 506.

In step 506 a value of an objective function is determined based on the global embeddings. In an example the objective function is indicative of an amount of agreement between the first global embedding, $e^1$, and the second global embedding, $e^2$. Or put in other words, in this example the objective function indicates how similar (or how close in the latent space) the first global embedding, $e^1$, is to the second global embedding, $e^2$. Since the first global embedding, $e^1$, and the second global embedding, $e^2$, are generated based on the same underlying data, the objective function will be maximised or minimised (depending on the specific implementation of the objective function) when the aggregator extracts high quality representations of the current state that are robust to missing modalities.

In other examples any self-supervised objective function that indicates the agreement between the first global embedding, $e^1$, and the second global embedding, $e^2$, can be used. After determining a value of the objective function in step 506, the method proceeds to step 507.

In step 507 the trainable weights of the second machine learning architecture 400 are updated based on the determined value of the objective function. In step 507, the weights associated with the feature extractors in the set of feature extractors 201 (e.g. the first set of weights, $W_1$, the second set of weights, $W_2$, the third set of weights, $W_3$, and the fourth set of weights, $W_4$) and the fifth set of weights, $W_5$, which is shared by the first aggregator 206 and the second aggregator 403 are updated based on the value of the objective function.

In an example the trainable weights are updated using backpropagation (i.e. backpropagation of errors). As known in the art, in this technique a partial derivate of the objective function with respect to each trainable weight is calculated. These partial derivatives are subsequently used to update the value of each trainable weight.

In an example where the aim is to minimise the objective function, the trainable weights in the second machine learning architecture 400 are updated using gradient decent such that:

$$w_n^{(i,j)} = w_n^{(i,j)} - \alpha \frac{dJ}{\partial w_n^{(i,j)}}$$

where:

$w_n^{(i,j)}$ is the trainable weight for the $i^{th}$ neuron in the $j^{th}$ layer of the $n^{th}$ set of trainable weights;

$\alpha$ is the learning rate. Optionally, the learning rate is predetermined; and

$\frac{dJ}{\partial w_n^{(i,j)}}$ is the partial derivative of the objective function, $J$, with respect to the trainable weight $w_n^{(i,j)}$.

In an example the partial derivate of the objective function, $J$, with respect to the trainable weight $w_n^{(i,j)}$ (i.e. $\frac{dJ}{\partial w_n^{(i,j)}}$ ) is determining using calculus (including using the chain rule) based on the structure of the machine learning models used in the second machine learning architecture 400 (e.g. based on the connection of the layers, the activation functions used by each neuron etc.). In another example, the partial derivate is determined using numerical methods (e.g. by numerically approximating the gradient with a finite difference approximation).

Although the above description describes one approach to modifying the trainable weights of the second machine learning architecture 400 with respect to the objective function it will be appreciated that other optimisation approaches could be used in other examples. Other approaches include, but are not limited to, "ADAM" gradient descent and "Momentum" gradient descent. In other examples, gradient ascent techniques are used when the aim is to maximise the objective function.

Returning to Fig. 5 after updating the trainable weights, the method proceeds to step 502 where training data is obtained again and the method is repeated. In an example, the mask used to generate the global representations in step 504 is regenerated for each training epoch (i.e. iteration through the batch/training set).

Optionally, the training method of Fig. 5 is repeated for a predetermined number of iterations. In other examples the training method of Fig. 5 is repeated until the objective function converges on a maximum or minimum value. In an example, the objective function is determined to have converged when the difference in the value of the objective function between training epochs (i.e. iterations of the method of Fig. 5) is less than a predetermined threshold.

The example method of Fig. 5 was discussed in relation to an example where a single training example is processed in each training iteration. The single training example in Fig. 5 comprises the first set of input data samples $X_1[1,2, ... , T_1]$, the second set of input data samples $X_2[1,2, ... , T_2]$, the third set of input data samples $X_3[1,2, ... , T_3]$, and the fourth set of input data samples $X_4[1,2, ...,T4]$.

In other examples a plurality of training examples are processed during each training iteration. In this example, step 502 comprises obtaining a plurality of training examples from the first set of sensors 101, steps 503 to 506 are repeated each example in the plurality of training examples, and the parameters are updated in step 507 based on a sum of the objective functions determined for each of the training examples. In an example, the masks used to generate the global representations in step 504 are the same for each training example in the plurality of training examples. As discussed above, the masks are updated (e.g. regenerated) after each training epoch (i.e. after completing step 507).

In an example where a plurality of training examples are processed in each training iteration, an objective function is used that: 1) encourages minimisation of the distance between embeddings of positive pairs; 2) encourages the reduction in the covariance of embeddings over the batch of training samples; and 3) maintains the variance of each variable of the embedding above a threshold.

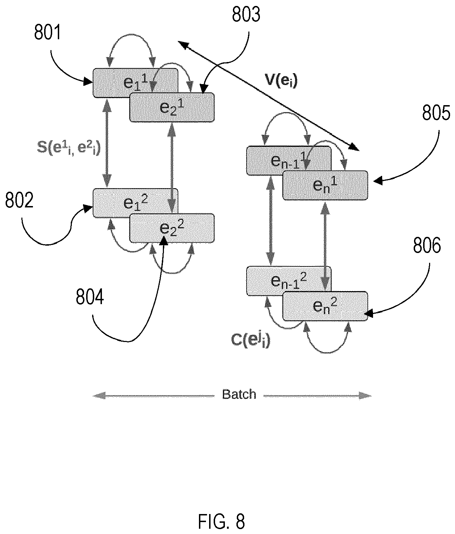

Fig. 8 shows an illustration of the terms used in an objective function according to an example. In particular, Fig. 8 shows a first global embedding for the first training sample in the batch 801, $e_1^1$ , a second global embedding for the first training sample in the batch 802, $e_1^2$ , a first global embedding for the second training sample in the batch 803, $e_2^1$ , a second global embedding for the second training sample in the batch 804, $e_2^2$ , a first global embedding for the $n^{th}$ training sample in the batch 805, $e_n^1$ , and a second global embedding for the $n^{th}$ training sample in the batch 806, $e_n^2$ .

In an example, the patches used to train the weights of the aggregators (e.g. the first aggregator 206 and the second aggregator 403) are represented by:

$$Z = [e_1^1, ..., e_n^1] \text{ and } Z' = [e_1^2, ..., e_n^2]$$

Where:

$n$ is the number of training examples in the set of training examples;

$e_n^1$ is the first global representation for the $n^{th}$ training example;

$e_n^2$ is the second global representation for the $n^{th}$ training example;

The objective function is calculated according to:

$$l(Z, Z') = \lambda s(Z, Z') + \mu[v(Z) + v(Z')] + \nu[c(Z) + c(Z')]$$

Where:

$s(Z, Z')$ is an invariance criterion between $Z$ and $Z'$;
$c(Z)$ is a covariance regularization term;
$v(Z)$ is a variance regularization term;
$\mu$ is a first hyperparameter;
$\lambda$ is a second hyperparameter;
$\nu$ is a third hyperparameter;

In an example, the invariance criterion $s(Z, Z')$ is calculated according to:

$$s(Z, Z') = \frac{1}{n} \sum_i \left\| e_i^1 - e_i^2 \right\|_2^2$$

Where:
$n$ is the number of training examples in the set of training examples;
In an example, the covariance regularisation term $c(Z)$ is calculated according to:

$$c(Z) = \frac{1}{d} \sum_{i \neq j} [C(Z)]_{i,j}^2$$

Where:

$d$ is the dimension number of the global embeddings; and
$C(Z)$ is the covariance matrix of $Z$.

In an example, the covariance matrix $C(Z)$ is calculated according to:

$$C(Z) = \frac{1}{n-1} \sum_{i=1}^{n} (e_i^1 - \overline{e^1})(e_i^1 - \overline{e^1})^T$$

Where:

$$\overline{e^1} = \frac{1}{n} \sum_{i=1}^{n} e_i^1$$

In an example, the variance regularization term $v(Z)$ is calculated according to:

$$v(Z) = \frac{1}{d} \sum_{j=1}^{d} \max(0, \gamma - S(z^j, \epsilon))$$

Where:

$\gamma$ is a target value for the standard deviation. Optionally, the target value is 1;

$S\left(z_j^1, \epsilon\right)$ is the regularized standard deviation of $z^j$;

$z^j$ is a vector comprising each value at dimension $j$ of all of the vectors in $Z$;

$\varepsilon$ is a predetermined small value to prevent numerical instability;

In an example, the regularized standard deviation is calculated according to:

$$S(x, \epsilon) = \sqrt{\mathrm{Var}(x) + \epsilon}$$

Where:

Var($x$) is the variance of the variable $x$.

As illustrated in Fig. 8, the objective function described above: 1) encourages a minimisation of the distance between embeddings of positive pairs (i.e. pairs of inputs that are formed by different data augmentations of the same input sample) as represented by the invariance term, $s(e_n^1, e_n^2)$; 2) encourages a reduction of the covariance over a batch to zero as represented by the covariance regularisation terms $c(Z)$ and $c(Z')$; and 3) maintains the variance of each variable of the embedding (over the batch) to be above a threshold as represented by the variance regularization terms $v(Z)$ and $v(Z')$.

Although one specific example of an objective function for processing a plurality of training examples is described above, it will be appreciated that other objective functions could be used in other examples.

Returning to Fig. 5. After completing the method of training in Fig. 5, each feature extractor in the set of feature extractors 201 has been trained and so has the first aggregator 206. In particular, each feature extractor in the set of feature extractors 201 has been trained to generate high-quality feature embeddings that are specific to each modality. Furthermore, the first aggregator 206 has learnt the multi-dimensional dependencies (e.g. the spatial and temporal dependencies) across the input data sources and has been trained to generate a representation of the current system state being observed by the sensors using a lower dimensional representation (thereby compressing the data from the first set of sensors 101).

Additionally, by removing local embeddings during patch selection (optionally, randomly) the first aggregator 206 learns to represent the current system state being observed by the sensors (i.e. generate a global embedding) in a way that is robust to missing modalities (i.e. in a way that is invariant to the presence of all modalities).

Finally, the method of training in Fig. 5 uses unlabelled data to train the modality-specific feature extractors and the first aggregator 206 in a way that is robust to missing modalities. Using unlabelled data for this training is advantageous because obtaining unlabelled data is often more practical and cost efficient than attempting to obtain labelled data.

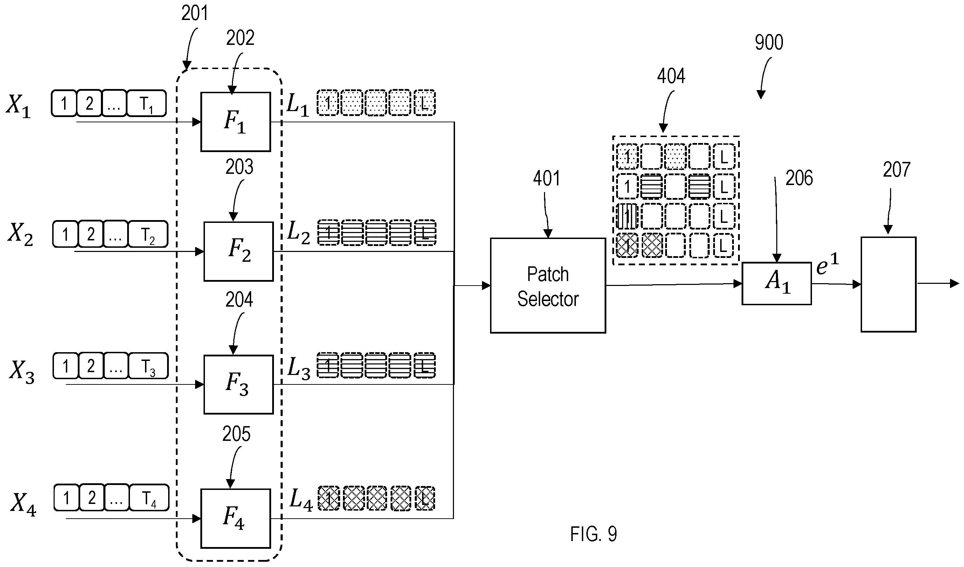

Fig. 9 shows a third machine learning architecture 900 used during supervised training according to an example. In particular, Fig. 9 shows a third machine learning architecture 900 that is used to train part of the first machine learning architecture 200. Those parts being the feature extractors in the set of feature extractors 201, the first aggregator 206, and the fourth machine learning model 207. In Fig. 9 same reference numerals as in Fig. 2 and Fig. 4 are used to represent same components with same functionality. As a result, a detailed discussion of their functionality will be omitted for the sake of brevity.

The third machine learning architecture 900 comprises the set of feature extractors 201, which comprises the first feature extractor 202, $F_1$, the second feature extractor 203, $F_2$, the third feature extractor 204, $F_3$, and the fourth feature extractor 205, $F_4$. The third machine learning architecture 900 further comprises: the first patch selector 401, the first aggregator 206 and the fourth machine learning model 207.

In the third machine learning architecture 900 the outputs of each feature extractor in the set of feature extractors 201 (i.e. the sets of local embeddings) are inputted into first patch selector 401. The output of the patch selector is a first global representation 404, wherein the first global representation 404 comprises some but not all of the local embeddings in the sets of local embeddings. The first global representation 404 is inputted into the first aggregator 206. The first aggregator 206 is configured to generate the first global embedding, $e^1$, based on the first global representation 404.

In the third machine learning architecture 900 of Fig. 9, the first global embedding, $e^1$, is provided as an input to the fourth machine learning model 207. The fourth machine learning model 207 is configured to generate a prediction/inference based on the first global embedding, $e^1$, and the sixth set of trainable weights, $W_6$. The output of the fourth machine learning model 207 comprises information indicating a prediction/inference for the particular task that the fourth machine learning model 207 is trained for. In an example where the fourth machine learning model 207 is configured to perform classification (e.g. determining whether or not a user has fallen over), the output comprises information identifying a class label (e.g. information indicating whether a user has fallen over).

Unlike the first method of training discussed above in Fig. 5, which trains the modality-specific feature extractors in the set of feature extractor 201 and the first aggregator 206 based on unlabelled data using self-supervised learning, the second method of training (discussed in more detail below) trains the modality-specific feature extractors in the set of feature extractors 201, the first aggregator 206 and the fourth machine learning model 207 based on labelled data using supervised learning.

These methods of training are introduced as separate methods. However, as will be discussed in more detail below, it is possible to combine both of these training methods into one process where, for example, the first method of training is used in a "general training" phase to train the feature extractors in the first set of feature extractors 201, and the aggregator using unlabelled data and the second method of training is used in a "fine training" phase to train the feature extractors, the aggregator and the classifier for a specific task.

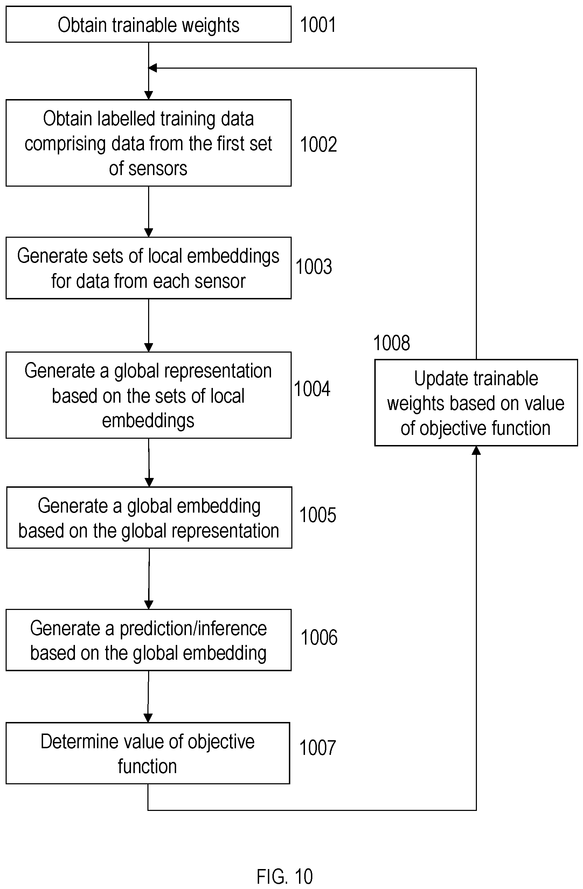

Fig. 10 shows a method of training a second part of the first machine learning architecture 200 according to an example. In particular, the method of Fig. 10 is used for training the feature extractors in the set of feature extractors 201, the first aggregator 206 and the fourth machine learning model 207. In this context, training means learning parameters/weights that could be used by the components during inference. The method begins in step 1001.

In step 1001 the trainable weights are obtained. The trainable weights in the example of Fig. 10 comprises: the weights of each feature extractor in the set of feature extractors 201 (e.g. $W_1$, $W_2$, $W_3$ and $W_4$), the weights used by the first aggregator 206 (e.g. the fifth set of weights, $W_5$) and the weights used by the fourth machine learning model 207 (e.g. the sixth set of weights, $W_6$). In an example, obtaining the weights used by the fourth machine learning model 207 comprises randomly initialising the sixth set of weights, $W_6$. After obtaining the trainable weights, the method proceeds to step 1002.

In step 1002 labelled training data is obtained. When used in the example system of Fig. 1, the labelled training data comprises at least: the first set of input data samples $X_1[1,2, ... , T_1]$ associated with the first sensor 102, the second set of input data samples $X_2[1,2, ... , T_2]$ associated with the second sensor 103, the third set of input data samples $X_3[1,2, ... , T_3]$ associated with the third sensor 104, the fourth set of input data samples $X_4[1,2, ... , T_4]$ associated with the fourth sensor 105, and a class label associated with the input data samples (e.g. whether the data indicates that the user is in the 'fallen over' class). Optionally, step 1002 also comprises augmenting the obtained set of input data samples using the same techniques as described in relation to step 502 of Fig. 5. The method proceeds to step 1003.

In step 1003 sets of local embeddings are generated for data from each of the sensors in the first set of sensors 101. The sets of local embeddings are generated in the same way as step 503 of Fig. 5. As a result, a detail discussion will be omitted for brevity. The method proceeds to step 1004.

In step 1004 a first global representation 404 is generated by discarding at least one embedding from the set of local embeddings. In an example, the first global representation 404 is generated by randomly discarding one or more local embeddings in the sets of the local embeddings. In an example the first global representation 404 is generated according to the methods of Fig. 6 or Fig. 7. After generating the first global representation 404, the method proceeds to step 1005.

In step 1005 a first global embedding is generated based on the first global representation 404. In particular, in step 1005 a first global embedding, $e^1$, is generated by inputting the first global representation 404 into the first aggregator 206 that is configured according to the fifth set of trainable weights, $W_5$. After obtaining the first global embedding, $e^1$, the method proceeds to step 1006.

In step 1006 a prediction/inference is generated based on the first global embedding, $e^1$. In particular, in step 1006 a prediction/inference is generated by inputting the first global embedding, $e^1$, into the fourth machine learning model 207 that is configured to generate an output prediction/inference based on the input and the sixth set of weights, $W_6$.

In an example where the fourth machine learning model 207 implements a classifier, the prediction/inference comprises information associated with a class label. In the specific example where the first machine learning architecture 200 is used for the task of predicting whether a user has fallen over, the prediction/inference generated by the fourth machine learning model 207 comprises an indication of whether the user has: A) fallen over, or B) not fallen over. The method proceeds to step 1007.

In step 1007 a value of an objective function is determined. In an example where the fourth machine learning model 207 is used for a classification task, the objective function is a classification cost function. In an example the objective function used in step 1007 is determined based on a difference between the information associated with the class label outputted by the fourth machine learning model 207 in step 1006 and the label associated with the training data obtained in step 1002. In a specific example, the objective function is the cross-entropy loss. The method proceeds to step 1008.

In step 1008, the trainable weights in the third machine learning architecture 900 are updated based on the value of the objective function. In particular, the first to sixth trainable weights ($W_1$, $W_2$, $W_3$, $W_4$, $W_5$, $W_6$) are updated with the aim of optimising (e.g. to minimise or to maximise) the objective function. In an example, the trainable weights are updated using the same techniques as described in relation to step 507 of Fig. 5. For example, by using gradient decent where the partial derivate of the objective function with respect to each trainable weight is determined analytically (e.g. from first principles based on the structure of machine learning models) or numerically.

After updating the trainable weights in step 1008 the method proceeds to step 1002 where the training method is repeated. In an example, steps 1002 - 1008 are repeated for a predetermined number of iterations. In another example steps 1002 - 1008 are repeating until the objective function converges (e.g. on a maximum or a minimum value).

Although the method of Fig. 10 was discussed in relation to a single training example, in other examples step 1002 comprises obtaining a batch of training data (comprising a plurality of training examples) and steps 1003 - 1007 are performed for each training example. In this example, the objective function calculated in step 1007 is based on the sum of the values for each training example.

After performing the method of Fig. 10, the fourth machine learning model 207 is trained to map the global embeddings to a prediction/inference (e.g. to a class label). Furthermore, in the method of training described in Fig. 10, the modality-specific feature extractors in the set of feature extractors 201, and the first aggregator 206 are further trained to extract useful features (i.e. generate a lower-dimensional representation of the input state) that is of use for the downstream task (e.g. classification). Finally, by introducing the patch selection step (i.e. discarding one or more local embeddings in the sets of local embeddings), the features being learnt are robust to missing modalities in use (thereby obtaining more accurate prediction in use).

There is also provided a method of deploying the first machine learning architecture 200 in the multi-modal machine learning system 100.

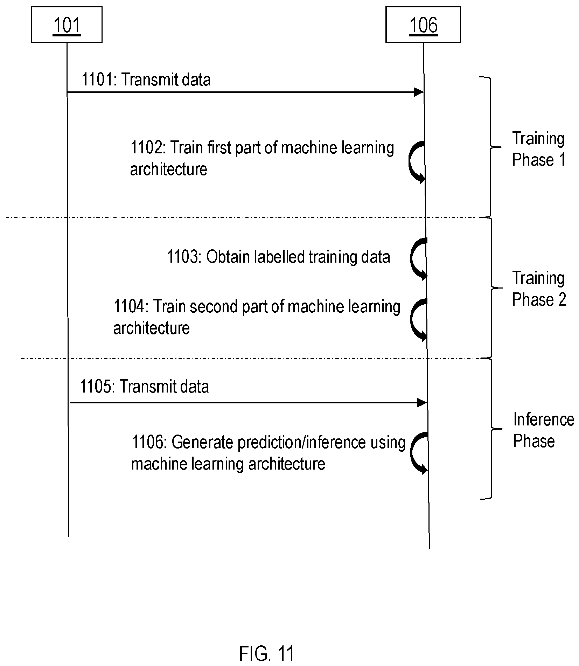

Fig. 11 shows a first method of deploying the first machine learning architecture 200 in the multi-modal machine learning system 100 according to an example. Fig. 11 uses same reference numerals as Fig. 1 to denote same components. As a result, a detailed discussion will be omitted for brevity. The method begins in step 1101.

In step 1101 the first set of sensors 101 transmit data to the first apparatus 106. In an example, the data comprises a first set of input data samples $X_1[1,2, ... , T_1]$, a second set of input data samples $X_2[1,2, ... , T_2]$, a third set of input data samples $X_3[1,2, ... , T_3]$, and a fourth set of input data samples $X_4[1,2, ... , T_4]$). The data obtained in step 1101 is unlabelled. The method proceeds to step 1102. In step 1102 the first apparatus trains a first part of the first machine learning architecture 200 using the method of training described in relation to Fig. 5.

In steps 1101 and 1102 the feature extractors in the first set of feature extractors 201, and the first aggregator 206 are trained using unlabelled training data. The combination of steps 1101 and 1102 is also referred to as the "Training Phase 1". The method proceeds to step 1103.

In step 1103 the first apparatus 106 obtains labelled training data. As discussed above, in an example the labelled training data comprises a first set of input data samples $X_1[1,2, ... , T_4]$, a second set of input data samples $X_2[1,2, ... , T_2]$, a third set of input data samples $X_3[1,2, ... , T_3]$, a fourth set of input data samples $X_4[1,2, ... , T_4]$, and a class label associated with the input data samples. In an example, the first-to-fourth set of input data samples used in step 1103 are different to the first-to-fourth set of input data used in step 1101. In an example, the labelled training data is retrieved from a separate entity (e.g. a server) that stores the data. The method proceeds to step 1104.

In step 1104 the first apparatus 106 trains a second part of the first machine learning architecture 200 using the method of training described in relation to Fig. 10. When obtaining the trainable weights in step 1001, the first apparatus 106 retrieves the trainable weights that were learnt in training phase 1 using unlabelled data (i.e. the weights obtained in step 1102) and randomly initialises the sixth set of weights, $W_6$, associated with the fourth machine learning model 207.

In steps 1103 and 1104 the modality-specific feature extractors in the first set of feature extractors 201, the first aggregator 206, and the fourth machine learning model 207 are trained using labelled training data. The combination of steps 1103 and 1104 is also referred to as the "Training Phase 2", or "fine-tuning". The method proceeds to step 1105.

In step 1105 the sensors in the first set of sensors 101 transmit data (e.g. while the sensors are being worn by a user) to the first apparatus 106. The method proceeds to step 1106. In step 1106 the first apparatus generates predictions/inferences using the method of inference as described in relation to Fig. 3. In this example, the weights for each feature extractor, the first aggregator and the fourth machine learning model 207 are those weights that were obtained by the first apparatus 106 after performing the method of training in step 1104. The combination of steps 1105 and 1106 is also referred to as the "Inference Phase". In an example the prediction/inference is output to a user. For example, by being displayed on a display contained in the first apparatus 106.

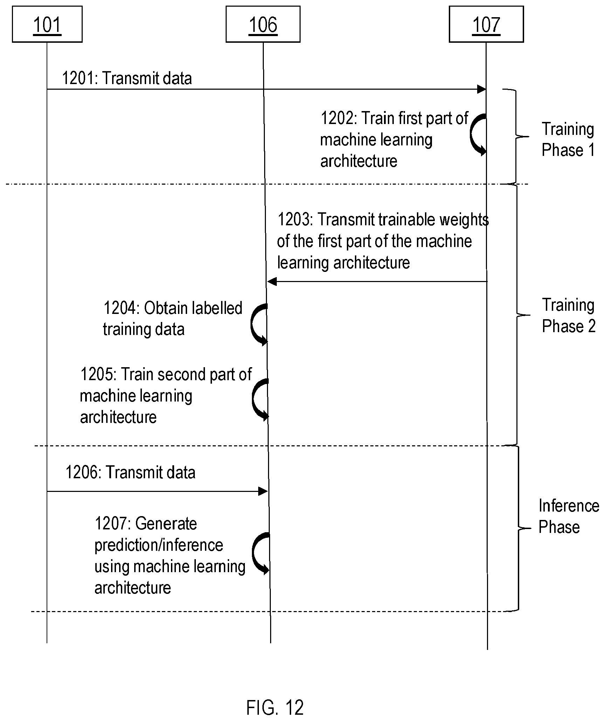

Fig. 12 shows a second method of deploying the first machine learning architecture 200 in the multi-modal machine learning system 100 according to an example. Fig. 12 uses same reference numerals as Fig. 1 to denote same components. As a result, a detailed discussion will be omitted for brevity. In the example of Fig. 12, the multi-modal machine learning system 100 comprises the second apparatus 107 (i.e. the server). The method begins in step 1201.

In step 1201 the first set of sensors 101 transmit data to the second apparatus 107 (e.g. the server). In an example, the data comprises a first set of input data samples $X_1[1,2, ... , T_1]$, a second set of input data samples $X_2[1,2, ... , T_2]$, a third set of input data samples $X_3[1,2, ... , T_3]$, and a fourth set of input data samples $X_4[1,2, ... , T4]$). The data obtained in step 1201 is unlabelled. The method proceeds to step 1202. In step 1202 the second apparatus 107 trains a first part of the first machine learning architecture 200 using the method of training described in relation to Fig. 5.

In steps 1201 and 1202 the modality-specific feature extractors in the first set of feature extractors 201, and the first aggregator 206 are trained by the second apparatus 107 using unlabelled training data. The combination of steps 1201 and 1202 is also referred to as the "Training Phase 1". The method proceeds to step 1203.

In step 1203, the second apparatus 107 transmits the trainable weights of the first part of the machine learning architecture (e.g. the first set of weights, $W_1$, the second set of weights, $W_2$, the third set of weights, $W_3$, the fourth set of weights, $W_4$ and the fifth set of weights, $W_5$) obtained in step 1202 to the first apparatus 106. The method proceeds to step 1204.

In step 1204 the first apparatus 106 obtains labelled training data. In an example the labelled training data comprises a first set of input data samples $X_1[1,2, ... , T_1]$, a second set of input data samples $X_2[1,2, ... , T_2]$, a third set of input data samples $X_3[1,2, ... , T_3]$, a fourth set of input data samples $X_4[1,2, ... , T_4]$, and a class label associated with the input data samples. In an example, the first-to-fourth set of input data samples used in step 1204 are different to the first-to-fourth set of input data used in step 1201. In an example, the labelled training data is retrieved from an entity (e.g. a server) that stored the data. In another example, the labelled training data is retrieved from the first apparatus 106 (e.g. from non-volatile storage). The method proceeds to step 1205.

In step 1205 the first apparatus 106 trains a second part of the first machine learning architecture 200 using the method of training described in relation to Fig. 10. When obtaining the trainable weights in step 1001, the first apparatus 106 uses the weights received in step 1203 and randomly initialises the sixth set of weights, $W_6$, associated with the fourth machine learning model 207.

In steps 1203, 1204, and 1205 the modality-specific feature extractors in the first set of feature extractors 201, the first aggregator 206, and the fourth machine learning model 207 are trained by the first apparatus 106 using labelled training data. The combination of steps 1203, 1204 and 1205 is also referred to as the "Training Phase 2". The method proceeds to step 1206.

In step 1206 the sensors in the first set of sensors 101 transmit data (e.g. while the sensors are being worn by a user) to the first apparatus 106. The method proceeds to step 1207. In step 1207 the first apparatus 106 generates predictions/inferences using the method of inference as described in relation to Fig. 3. In this example, the weights for each feature extractor, the first aggregator and the fourth machine learning model are those weights that were obtained by the first apparatus 106 after performing the method of training in step 1205. The combination of steps 1206 and 1207 is also referred to as the "Inference Phase". In an example the prediction/inference generated in step 106 is transmitted to an external entity (e.g. to the second apparatus 107). In another example the generated prediction/inference is displayed (e.g. on a display of the first apparatus 106).

In the examples above, the input data samples are discussed in relation to an example where the first set of input data samples comprises $T_1$ data samples, the second set of input data samples comprises $T_2$ data samples, the third set of input data samples comprises $T_3$ data samples, and the fourth set of input data samples comprises $T_4$ data samples. In this case, the samples are a function of time (i.e. each sample in the set of input data samples is measured/observed at a different time). However, for the avoidance of any doubt, it is emphasized that the input data samples could contain other data types. In one example, one of the sets of input data samples comprises frequency data (e.g. measurements/observations that are a function of frequency). In another example, one of the sets of input data samples comprises spatial data (e.g. image data comprising measurement/observations that are a function of position, specifically pixel position). In an example, the first set of input data samples and the second set of input data samples comprises data of different types.

In the above description, reference is made to the "temporal" direction and the "spatial" direction when discussing the global representation. As discussed above, relationships in the "spatial" direction refer to relationships in the data from different sensors for a given local embedding sample number. Relationships in the "temporal" direction refer to relationships in the data that are a function of time, for a given input sensor. In the case that the input data does not correspond to time samples (e.g. the input data corresponds to spatial data such as pixel values) the "temporal" direction refers to the direction of the local embedding sample number. In this case relationships in the "temporal" direction relate to relationships between different local embeddings for a given sensor input (e.g. between L1[1], $L_1[2]$, L1[3], etc.).

The above methods are discussed in relation to an example where the first set of sensors 101 comprises four sensors with specific data types (e.g. audio, heart rate etc.). However, it is emphasized for the avoidance of any doubt, that a different number of sensors with different data types could be used in other example implementations.

Furthermore, in the above examples, the input data (e.g. the first set of input data samples, $X_1[1,2, ... , T_1]$, the second set of input data samples, $X_2[1,2, ... , T_2]$, the third set of input data samples $X_3[1,2, ..., T_3]$, and the fourth set of data samples $X_4[1,2, ..., T_4]$) are associated with sensor data. In other examples, one or more of the sets of input data samples are not associated measurements/observations made by a sensor. For example, in one example, one of the input data samples comprises synthetically generated data that is not associated with the measurements/observations of a physical sensor.

In the examples above, the sets of input data samples have a specified length. For example, the first set of input data samples has length $T_1$, the second set of data samples has length $T_2$, the third set of data samples has length $T_3$, and the fourth set of data samples has length $T_4$. In an example, the sets of input data samples have a length greater than or equal to 1 (i.e. $T_1 \geq 1$, $T_2 \geq 1$, etc.). In an example, the first set of input data samples is referred to as a first data sample, and the second set of input data samples is referred to as a second data sample etc.

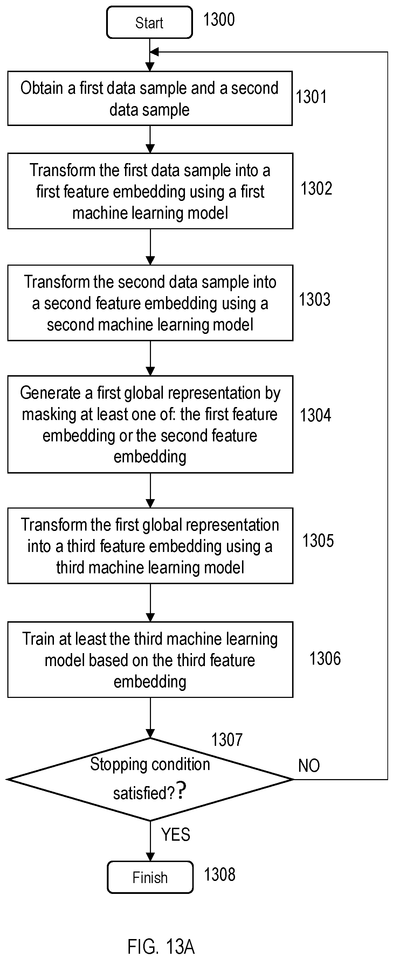

Fig. 13A shows a method of training at least the third machine learning model according to an example. As discussed above, in an example the first aggregator 206 comprises the third machine learning model. The method begins in step 1300 and proceeds to step 1301.

In step 1301, a first data sample and a second data sample are obtained. In one example step 1301 comprises performing step 502 of Fig. 5 (i.e. obtaining unlabelled data). In another example step 1302 comprises performing step 1002 of Fig. 10 (i.e. obtaining labelled data). The method proceeds to step 1302.

In step 1302, the first data sample is transformed into a first feature embedding using a first machine learning model. The method proceeds to step 1303. In step 1303 the second data sample is transformed into a second feature embedding using a second machine learning model. In an example, steps 1302 and 1303 comprise performing step 503 of Fig. 5. In another example, steps 1302 and 1303 comprise performing step 1003 of Fig. 10. The method proceeds step 1304.

In step 1304, a first global representation is generated by masking at least one of: the first feature embedding or the second feature embedding. In example step 1304 comprises performing step 504 of Fig. 5. In another example, step 1304 comprises performing step 1004 of Fig. 10. The method proceeds to step 1305.

In step 1305 the first global representation is transformed into a third feature embedding using a third machine learning model. In an example the third feature embedding is a first global embedding. In an example step 1305 comprises performing step 505 of Fig. 5. In another example step 1305 comprises performing step 1005 of Fig. 10. The method proceeds to step 1306.

In step 1306, at least the third machine learning model is trained based on the third feature embedding. In an example step 1306 comprises performing steps 506 and 507 of Fig. 5. In another example step 1306 comprises performing steps 1006, 1007 and 1008 of Fig. 10. The method proceeds to step 1307.

In step 1307 it is determined whether a stopping condition is met. In an example the stopping condition is whether the training method (i.e. steps 1301 - 1306) has been executed at least predetermined number of times. In another example, the stopping condition is met when a difference in a value of an objective function between successive training iterations is less than a threshold.

In response to the determining that the stopping condition has been met, the method proceeds to step 1308 where the method finished. In response to determining that the stopping condition has not been met, the method proceeds to step 1301 where the method is repeated.

The methods described herein were evaluated with a test dataset. In an example, the first machine learning architecture 200 is configured for the task of physical activity monitoring, where the fourth machine learning model 207 is configured to classify the activity being performed by a user wearing a plurality of sensors. In this example, the first set of sensors 101 comprises at least 3 inertial measurement units (IMU), wherein a first inertial measurement unit (IMU) is worn over the wrist on the dominant arm, a second inertial measurement unit (IMU) is worn on the chest, and a third inertial measurement unit (IMU) is worn on the dominant side's ankle. In this example, the fourth machine learning model 207 comprises a classifier with at least the following output classes: sitting, standing, walking, running, cycling, nordic walking, ascending/descending stairs, rope-jumping, other.

In an example the test dataset is the "PAMAP2" data set available from "Dua, D. and Graff, C. (2019). UCI Machine Learning Repository [http://archive.ics.uci.edu/ml], Irvine, CA: University of California, School of Information and Computer Science", which is incorporated herein by reference.

In order to compare the methods described herein three different approaches were compared. A first fully supervised approach (referred to as "Supervised") was implemented, where the machine learning architecture (i.e. the feature extractors, the aggregator and the classifier) were trained end-to-end using only labelled data.

A second approach (referred to as "SSL") was also implemented. In the "SSL" baseline, the feature extractors and the aggregator are trained using self-supervised learning and masking (e.g. according to the method of Fig. 5), and then the classifier only is trained using labelled data.

A third approach (referred to as "Fine tuned") was also implemented. In the "Fine tuned" approach the feature extractors and the aggregator are first trained using self-supervised learning and masking (e.g. according to the method of Fig. 5), and then the whole machine learning architecture (i.e. the feature extractors, the aggregator and the classifier) are retrained or fine-tuned based on labelled data (e.g. according to the method of Fig. 10).

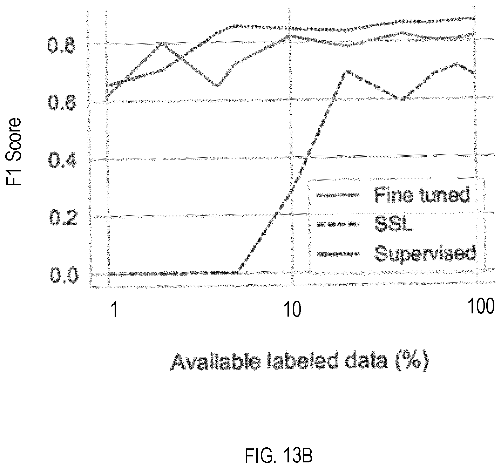

Fig. 13B shows a performance comparison according to an example. In particular, Fig. 13B shows a comparison of the F1 score for a test data set achieved by using the "supervised", "SSL", and "fine tuned" approaches described above. The results were obtained using random masking (where appropriate) and using a batch size of 8.

The vertical axis labelled "F1 score" is the F1 score (i.e. a metric that combines the precision and recall of a machine learning model) for a given test data set. The horizontal axis, labelled "Available labelled data" shows an amount of labelled data from a training data set that was used to further train the machine learning architecture. For example, Available labels = 10% corresponds to a test setup where: 1) in the "Supervised" approach, the machine learning architecture is trained end-to-end with 10% of the available labelled data; 2) in the "SSL" approach, the feature extractors and the aggregator are trained using self-supervised learning and masking (e.g. as in Fig. 5) and then the classifier only is trained using 10% of the available labelled data; and 3) in the "Fine tuned" approach the feature extractors and the aggregator are trained using self-supervised learning and masking (e.g. as in Fig. 5) and then the whole of the machine learning architecture (i.e. the feature extractors, the aggregator and the classifier) are retrained or fine tuned using 10% of the available labelled data.

As can be seen in Fig. 13B, even with small amounts of labelled data, the "Fine tuned" approach described herein achieves performance on par with supervised models. Furthermore, the "SSL" approach can achieve similar performance with supervised models when the classifier is trained using more data. However, it will be appreciated that in use, the "Fine tuned" and "SSL" approaches are more robust to missing modalities.

As discussed above, the first machine learning architecture 200 uses a plurality of machine learning models. For example, the first feature extractor 202, $F_1$, is implemented using a first machine learning model, the second feature extractor 203, $F_2$, is implemented using a second machine learning model, the first aggregator 206 is implemented using a third machine learning model and a classifier/regressor is implemented using the fourth machine learning model 207. Various different types of machine learning model could be used to implement these functional blocks/components.

In an example, the first feature extractor 202, $F_1$, is implemented using a sequence model. In an example, the first feature extractor 202, $F_1$, is implemented using a Recurrent Neural Network (RNN). As known in the art, a Recurrent Neural Network (RNN) is a stateful neural network, which means that it not only retains information from the previous layer but also from the previous pass. In the Recurrent Neural Network (RNN) connections between nodes can create a cycle, allowing the output from some nodes to affect subsequent input to the same nodes. This allows the machine learning model to exhibit temporal dynamic behaviour. In an example, at least one of the feature extractors is a many-to-many RNN where the number of input samples (e.g. $T_1$) does not equal the number of output samples ($L$). Advantageously, the use of a many-to-many RNN enables a variable length input (e.g. $T_1$, $T_2$ etc.) to be converted into a fixed size (e.g. $L$) feature embedding.

In an example, another feature extractor from the first set of feature extractors is implemented using a Convolutional Neural Network (CNN). As known in the art a Convolutional Neural Network (CNN) is an artificial Neural Network comprising convolutional layers, where parts of an input are convolved with a filter to generate feature maps. Advantageously, using a Convolutional Neural Network (CNN) enables spatial features to be extracted from the input data samples.

In other examples, one or more of the machine learning models are implemented using fully connected (artificial) neural networks.

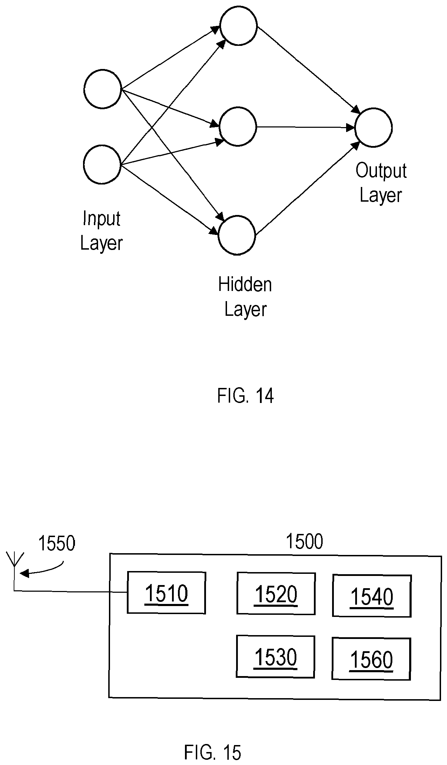

Fig. 14 shows an illustration of a fully connected (artificial) Neural network according to an example. In particular, Fig. 14 shows an (artificial) neural network comprising an input layer, a hidden layer and an output layer. In the example of Fig. 14, the input layer comprises two neurons, the hidden layer comprises three neurons and the output layer comprises a single neuron. Although one example implementation is shown in Fig. 14, it will be appreciated that other implementations may use a different number of neurons per layer and a different number of hidden layers. In the (artificial) neural network the output from each neuron is: a weighted sum of the inputs, that is subsequently passed through an activation function (e.g. Sigmoid, ReLu, Tanh etc.). The weights of the weighted sum are trainable and are referred to as the trainable weights of the machine learning model. By training the weights of the machine learning model it is possible to implement a mathematical transform that maps a set of inputs to a specific set of outputs.

As can be seen from the description above, the above-described methods can be used to train feature extractors and an aggregator to generate embedding of the input data that are robust to missing data at the input. Generating representations that accurately reflect the state of the system being observed, even while missing input data modalities, enables improved performance from machine learning systems that subsequently use the global embedding for prediction/inference tasks. As will be appreciated, feature extraction is a form of data compression in the sense that the feature extractors are configured to represent the input data in a more compact representation for use in subsequent processing. In this way, the above-described methods could be described as a method of data compression where the transforms used for the compression are trained (or learnt) such that the resulting compressed data accurately reflects the state of the system being observed/measured even in the case that some of the input data is missing.

In the above description the methods are introduced in relation to an example where various sensors are used for the task of determining whether a user (wearing the sensors) has fallen over. However, for the avoidance of any doubt, it is emphasized that the methods can be used in other applications.

In an example, the first machine learning architecture 200 is used for the task of medical diagnosis. In this example the sets of input samples comprises image data (e.g. MRI image data) and text data (e.g. comprising test results, vital signs, patient demographics etc.). In this example, the fourth machine learning model 207 is configured to predict whether or not a patient has a medical disease (e.g. a cardiovascular disease).

In another example, the first machine learning architecture 200 is used for the task of activity tracking. In this example, the sets of input samples comprises accelerometer, gyroscopic data and heart rate data. In this example, the fourth machine learning model 207 is configured to predict the activity being performed by a user (e.g. one or more of: 'transient', 'lying', 'sitting', 'standing', 'walking', 'running', 'cycling', 'Nordic_walking', 'watching_TV', 'computer_work', 'car driving', 'ascending_stairs', 'descending_stairs', 'vacuum_cleaning', 'ironing', 'folding_laundry', 'house_cleaning', 'playing_soccer', and 'rope_jumping').

In another example, the first machine learning architecture 200 is used for the purpose of sleep detection. In this example, the sets of input samples comprise electroencephalogram (EEG), electrooculography (EOG), and chin electromyography (EMG) data and the fourth machine learning model 207 is configured to determine the phase of sleep of the user (e.g. Awake, Rapid Eye Movement, N1, N2-N3, and N4).

In another example, the first machine learning architecture 200 is used for the task of industrial process monitoring. In this example the sets of input samples comprise image data (e.g. of an object being manufactured) and process information (e.g. temperature data). In this example, the fourth machine learning model 207 is configured to predict whether or not an object being manufactured is defective.

In another example, the first machine learning architecture 200 is used for the task of monitoring critical infrastructure (e.g. a bridge). In this example the sets of input samples comprise image data (e.g. of a part of the bridge) and other time-series data (e.g. weather readings). In this example, the fourth machine learning model 207 is configured to predict whether or not a part of the critical infrastructure being monitored needs to be repaired.

In another example, the first machine learning architecture 200 is used for the task of object detection (specifically person identification). In this example the sets of input samples comprise image data (e.g. corresponding to a previous picture of the person of interest) and text information (e.g. comprising a textual description of the person of interest). In this example, the fourth machine learning model 207 is configured to predict whether or not an identified person is the person of interest.

Fig. 15 shows an implementation of the first apparatus according to an example. The first apparatus 1500 comprises an input/output module 1510, a processor 1520, a non-volatile memory 1530 and a volatile memory 1540 (e.g. a RAM). The input/output module 1510 is communicatively connected to an antenna 1550. The antenna 1550 is configured to receive wireless signals from, and transmit wireless signals to, other apparatuses (including, but not limited to, the second apparatus (e.g. the server) and the sensors in the first set of sensors 101). The processor 1520 is coupled to the input/output module 1510, the non-volatile memory 1530 and the volatile memory 1540.

The non-volatile memory 1530 stores computer program instructions that, when executed by the processor 1520, cause the processor 1520 to execute program steps that implement the functionality of a first apparatus as described in the above-methods. In an example, the computer program instructions are transferred from the non-volatile memory 1530 to the volatile memory 1540 prior to being executed. Optionally, the first apparatus also comprises a display 1560.

In an example, the non-transitory memory (e.g. the non-volatile memory 1530 and/or the volatile memory 1540) comprises computer program instructions that, when executed, perform the methods of any one of: Fig. 3; Fig. 5; Fig. 10; steps 1102 - 1106 of Fig. 11; steps 1204, 1205 and 1207 of Fig. 12; and/or Fig. 13A.

Whilst in the example described above the antenna 1550 is shown to be situated outside of, but connected to, the first apparatus 1500 it will be appreciated that in other examples the antenna 1550 forms part of the apparatus 1500.

In an example the second apparatus (e.g. the server) comprises the same components (e.g. an input/output module 1510, a processor 1520, a non-volatile memory 1530 and a volatile memory 1540 (e.g. a RAM)) as the first apparatus 1500. In this example, the non-volatile memory 1530 stores computer program instructions that, when executed by the processor 1520, cause the processor 1520 to execute program steps that implement the functionality of a second apparatus as described in the above-methods.

In an example, the non-transitory memory (e.g. the non-volatile memory 1530 and/or the volatile memory) comprises computer program instructions that, when executed, perform the methods of any one of: Fig. 5, Fig. 10, and/or step 1202 of Fig. 12.

The term "non-transitory" as used herein, is a limitation of the medium itself (i.e., tangible, not a signal) as opposed to a limitation on data storage persistency (e.g., RAM vs. ROM).

As used herein, "at least one of the following: <a list of two or more elements>" and "at least one of: <a list of two or more elements>" and similar wording, where the list of two or more elements are joined by "and" or "or", mean at least any one of the elements, or at least any two or more of the elements, or at least all the elements.

While certain arrangements have been described, the arrangements have been presented by way of example only and are not intended to limit the scope of protection. The concepts described herein may be implemented in a variety of other forms. In addition, various omissions, substitutions and changes to the specific implementations described herein may be made without departing from the scope of protection defined in the following claims.

**Claims**

1. Apparatus comprising means for:

   obtaining a first data sample and a second data sample;
   transforming the first data sample into a first feature embedding using a first machine learning model;
   transforming the second data sample into a second feature embedding using a second machine learning model;
   generating a first global representation by masking at least one of: the first feature embedding or the second feature embedding;
   transforming the first global representation into a third feature embedding using a third machine learning model; and
   training at least the third machine learning model based on the third feature embedding.

2. The apparatus according to claim 1, wherein training at least the third machine learning model based on the third feature embedding comprises:
   training the first machine learning model, the second machine learning model, and the third machine learning model based on the third feature embedding.

3. The apparatus according to any preceding claim, wherein the first data sample is associated with a first sensor and the second data sample is associated with a second sensor.

4. The apparatus according to any preceding claim, wherein the first data sample comprises a first plurality of data samples, the second data sample comprises a second plurality of data samples, the first feature embedding comprises a first plurality of feature embeddings, the second feature embedding comprises a second plurality of feature embeddings; and wherein:
   generating the first global representation by masking at least one of: the first feature embedding or the second feature embedding comprises:
   masking at least one feature embedding in the combination of the first plurality of feature embeddings and the second plurality of feature embeddings.

5. The apparatus according to claim 4, wherein generating the first global representation by masking at least one feature embedding in the combination of the first plurality of feature embeddings and the second plurality of feature embeddings, comprises:

   obtaining a threshold value;
   generating a random number; and
   determining if the random number is greater than the threshold value; and
   masking a first embedding in the first plurality of feature embeddings in response to determining that the random number is less than the threshold value.

6. The apparatus according to claim 4, wherein generating the first global representation by masking at least one feature embedding in the combination of the first plurality of feature embeddings and the second plurality of feature embeddings, comprises:

   determining a pivot location;
   determining a position value by sampling from a probability distribution, wherein the mean of the probability distribution is the pivot location; and
   adding a first embedding from the first plurality of feature embeddings to the first global representation based on the position value.

7. The apparatus according to any preceding claim, wherein the means are further configured for:

generating a second global representation by masking at least one of: the first feature embedding or the second feature embedding;

transforming the second global representation into a fourth feature embedding using the third machine learning model; and wherein:

training at least the third machine learning model based on the third feature embedding comprises:

training at least the third machine learning model based on the third feature embedding and the fourth feature embedding.

8. The apparatus according to claim 7, when dependent on claim 6, wherein generating the second global representation by masking at least one of: the first feature embedding or the second feature embedding comprises:

obtaining the pivot location;

determining a second position value by sampling from the probability distribution; and

adding a second embedding from the first plurality of feature embeddings to the second global representation based on the second position value.

9. The apparatus according to claim 7 or 8, wherein training at least the third machine learning model based on the third feature embedding and the fourth feature embedding comprises:

determining a value of a first objective function, wherein the first objective function indicates a similarity between the third feature embedding and the fourth feature embedding; and

training at least the third machine learning model based on the value of the first objective function.

10. The apparatus according to any preceding claim, wherein training at least the third machine learning model based on the third feature embedding comprises:

generating a first prediction using a fourth machine learning model and the first global representation;

obtaining a second value associated with the first data sample and the second data sample;

determining a value of a second objective function based on the first prediction and the second value; and

training at least the third machine learning model based on the value of the second objective function.

11. The apparatus according to claim 10, wherein training at least the third machine learning model based on the value of the second objective function comprises:

training the first machine learning model, the second machine learning model, the third machine learning model and the fourth machine learning model based on the value of the second objective function.

12. The apparatus according to any preceding claim, wherein the means are further configured for:

obtaining a third data sample and a fourth data sample;

transforming the third data sample into a fifth feature embedding using the first machine learning model;

transforming the fourth data sample into a sixth feature embedding using the second machine learning model;

generating a third global representation by combining the fifth feature embedding and the sixth feature embedding; and

transforming the third global representation into a seventh feature embedding using the third machine learning model.

13. The apparatus according to claim 12, when dependent on claims 10 or 11, wherein the means are further configured for:

generating a second prediction using the fourth machine learning model and the third global representation.

14. A method comprising:

obtaining a first data sample and a second data sample;

transforming the first data sample into a first feature embedding using a first machine learning model;

transforming the second data sample into a second feature embedding using a second machine learning model;

generating a first global representation by masking at least one of: the first feature embedding or the second feature embedding;

transforming the first global representation into a third feature embedding using a third machine learning model;

and
training at least the third machine learning model based on the third feature embedding.

15. A computer program comprising instructions which, when executed by an apparatus, cause the apparatus to perform at least the following:

obtaining a first data sample and a second data sample;
transforming the first data sample into a first feature embedding using a first machine learning model;
transforming the second data sample into a second feature embedding using a second machine learning model;
generating a first global representation by masking at least one of: the first feature embedding or the second feature embedding;
transforming the first global representation into a third feature embedding using a third machine learning model; and
training at least the third machine learning model based on the third feature embedding.

FIG. 1

FIG. 2

Obtain weights for each feature extractor, the first aggregator, and the fourth machine learning model — 301

Obtain data from the first set of sensors — 302

Generate sets of local embeddings for data from each sensor — 303

Form a global representation based on the sets of local embeddings — 304

Generate a global embedding based on the global representation — 305

Generate an inference using the fourth machine learning model — 306

FIG. 3

FIG. 4

```
┌─────────────────────────────┐
│  Initialise trainable weights │ 501
└─────────────────────────────┘
              │
              ▼
┌─────────────────────────────┐
│ Obtain unlabelled data comprising │ 502
│  data from the first set of sensors │
└─────────────────────────────┘
              │
              ▼
┌─────────────────────────────┐
│ Generate sets of local embeddings │ 503
│    for data from each sensor    │
└─────────────────────────────┘
              │
              ▼
┌─────────────────────────────┐
│ Generate global representations │ 504
│   based on the sets of local   │
│         embeddings           │
└─────────────────────────────┘
              │
              ▼
┌─────────────────────────────┐        507
│  Generate global embeddings   │     ┌─────────────────────┐
│     based on the global      │ 505 │  Update trainable    │
│       representations        │     │ weights based on value │
└─────────────────────────────┘     │  of objective function │
              │                      └─────────────────────┘
              ▼                              ▲
┌─────────────────────────────┐             │
│  Determine value of objective │ 506        │
│          function            │            │
└─────────────────────────────┘            │
              │                             │
              └─────────────────────────────┘
```

FIG. 5

| | | | | |
|---|---|---|---|---|
| 1 | | | | L |
| 1 | | | | L |
| 1 | | | | L |
| 1 | | | | L |

651

652

| | | | | |
|---|---|---|---|---|
| 0.48 | 0.70 | 0.49 | 0.06 | 0.34 |
| 0.64 | 0.47 | 0.45 | 0.58 | 0.86 |
| 0.87 | 0.89 | 0.61 | 0.09 | 0.67 |
| 0.95 | 0.45 | 0.02 | 0.58 | 0.22 |

| | | | | |
|---|---|---|---|---|
| 0 | 1 | 0 | 0 | 0 |
| 1 | 0 | 0 | 1 | 1 |
| 1 | 1 | 1 | 0 | 1 |
| 1 | 0 | 0 | 1 | 0 |

654     653

| | | | | |
|---|---|---|---|---|
| 1 | | | | L |
| 1 | | | | L |
| 1 | | | | L |
| 1 | | | | L |

**Flowchart:**

- Obtain sets of local embeddings — 601
- Obtain masking rate — 602
- Generate a vector of random numbers — 603
- Generate mask based on masking rate and vector of random numbers — 604
- Apply mask to sets of local embeddings — 605

FIG. 6

Obtain sets of local
embeddings

701

751

Obtain masking rate

702

Obtain pivot locations

703

752

pivot$_1$

pivot$_2$

704

Obtain a set of
embeddings

pivot$_1$

pivot$_2$

753

705

Select embeddings by
sampling a distribution
centered on each pivot

pivot$_1$

pivot$_2$

706

All sets
masked?

NO

754

YES

707

Finish

755

FIG. 7A

pivot$_1$

pivot$_2$

FIG. 7B

FIG. 8

FIG. 9

Obtain trainable weights | 1001

Obtain labelled training data comprising data from the first set of sensors | 1002

Generate sets of local embeddings for data from each sensor | 1003

Generate a global representation based on the sets of local embeddings | 1004

Generate a global embedding based on the global representation | 1005

Generate a prediction/inference based on the global embedding | 1006

Determine value of objective function | 1007

1008

Update trainable weights based on value of objective function

FIG. 10

FIG. 11

| 101 | 106 | 107 |
|-----|-----|-----|

1201: Transmit data

1202: Train first part of machine learning architecture

Training Phase 1

1203: Transmit trainable weights of the first part of the machine learning architecture

1204: Obtain labelled training data

1205: Train second part of machine learning architecture

Training Phase 2

1206: Transmit data

1207: Generate prediction/inference using machine learning architecture

Inference Phase

FIG. 12

Start — 1300

Obtain a first data sample and a second data sample — 1301

Transform the first data sample into a first feature embedding using a first machine learning model — 1302

Transform the second data sample into a second feature embedding using a second machine learning model — 1303

Generate a first global representation by masking at least one of: the first feature embedding or the second feature embedding — 1304

Transform the first global representation into a third feature embedding using a third machine learning model — 1305

Train at least the third machine learning model based on the third feature embedding — 1306

1307
Stopping condition satisfied? — NO

YES

Finish — 1308

FIG. 13A

FIG. 13B

FIG. 14

FIG. 15

Europäisches
Patentamt

European
Patent Office

Office européen
des brevets

## EUROPEAN SEARCH REPORT

Application Number

EP 23 15 6528

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Natalia Neverova: "Deep learning for human motion analysis", , 8 April 2016 (2016-04-08), XP055725540, Retrieved from the Internet: URL:https://tel.archives-ouvertes.fr/tel-0 1470466v2/document [retrieved on 2020-08-27] * the whole document * ----- | 1-15 | INV. G06F16/906 G16H50/20 G06N3/044 G06N3/084 G06N3/0895 G06N3/09 |
| | | | TECHNICAL FIELDS SEARCHED (IPC) |
| | | | G16H G06F G06N |

The present search report has been drawn up for all claims

| Place of search | Date of completion of the search | Examiner |
|---|---|---|
| The Hague | 5 July 2023 | Ntarlagiannis, V |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.......................................................................
& : member of the same patent family, corresponding
   document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **DUA, D. ; GRAFF, C.** CA: University of California, School of Information and Computer Science. *UCI Machine Learning Repository,* 2019, http://archive.ics.uci.edu/ml